# EUROPEAN PATENT APPLICATION

(11) **EP 2 050 764 A1**
(43) Date of publication of application: **22.04.2009**
(21) Application number: 07291259.5
(22) Date of filing: 15.10.2007
(51) Int. Cl.: C07K 16/46

(54) **Novel polyvalent bispecific antibody format and uses thereof**

(71) Applicant: sanofi-aventis, 75013 Paris (FR)
(72) Inventor: The designation of the inventor has not yet been filed
(74) Representative: Gruber, Daniel

(57) **Abstract**

The present invention relates to a polyvalent bispecific antibody format which comprises a first polypeptide which is a constant heavy chain and a second polypeptide which is a constant light chain, wherein the first polypeptide is attached to two or more variable domains of a heavy chain and the second polypeptide is attached to two or more variable domains of a light chain. A preferred embodiment of the present invention refers to a tetravalent bispecific tandem immunoglobulin (TBTI).
The polyvalent bispecific antibodies of the present invention resemble and keep the stability characteristics of an immunoglobulin molecule, expressing the heavy and light chains on two different polypeptides and incorporating the constant heavy regions and the constant light regions in order to stabilize the interaction between the variable heavy and the variable light domains and to force correct pairing of the corresponding VHNL. The present invention further refers to the uses of such polyvalent bispecific antibodies.

## Description

### FIELD OF THE INVENTION

The present invention relates to a polyvalent bispecific antibody format which contains several heavy chain variable domains and respectively light chain variable domains in tandem, in particular a tetravalent bispecific tandem immunoglobulin (TBTI). These polyvalent bispecific antibody formats resemble and keep the stability characteristics of an immunoglobulin molecule, expressing the heavy and light chains on two different polypeptides and incorporating the constant heavy regions and the constant light regions in order to stabilize the interaction between the variable heavy and the variable light domains and to force correct pairing of the corresponding VH/VL. The present invention further refers to the uses of such a polyvalent bispecific antibody.

### BACKGROUND OF THE INVENTION

### 1. Monospecific antibodies

### Structure of a natural antibody molecule

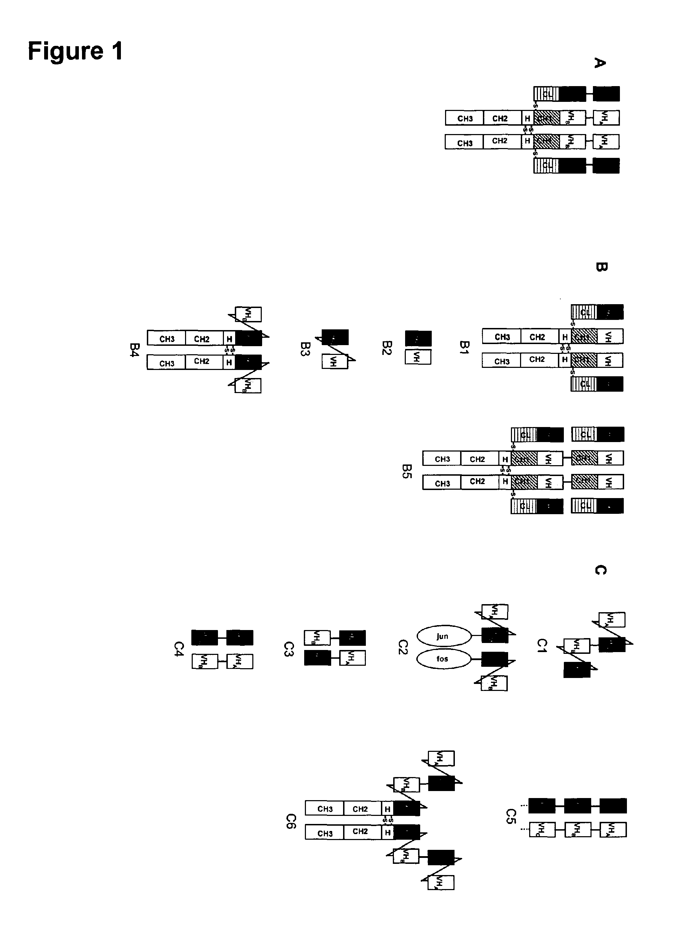

There has been much interest in recent years in antibodies and their fragments. It is well known that complete antibody molecules are Y-shaped molecules comprising four polypeptide chains: two heavy chains and two light chains (Fig. 1A). Each light chain consists of two domains, the N-terminal domain being known as the variable or VL domain and the C-terminal domain being known as the constant or CL domain (constant kappa (C*k*) or constant lambda (C□) domain). Each heavy chain consists of four or five domains, depending on the class of the antibody. The N-terminal domain is known as the variable or VH domain. The next domain is known as the first constant or CH1 domain. The next part of each heavy chain is known as the hinge region and this is then followed by the second, third and, in some cases, fourth constant or CH2, CH3 and CH4 domains respectively.

In an assembled antibody, the VL and VH domains associate together to form an antigen binding site. Also the CL and CH1 domains associate together to keep one heavy chain associated with one light chain. The two heavy-light chain heterodimers associate together partly by interaction of the CH2, CH3 and CH4 domains of the two heavy chains and partly because of interaction between the hinge regions on the two heavy chains.
Each heavy chain hinge region includes at least one, and often several, cysteine residues. In the assembled antibody, the cysteine residues in the heavy chains are aligned so that disulfide bonds can be formed between the cysteine residues in the hinge regions covalently bonding the two heavy-light chain heterodimers together (Fig. 1B₁).
Thus, fully assembled antibodies are monospecific in that they bind one antigen type and bivalent in that they have two independent antigen binding sites.

### Monospecific monovalent antibody fragments

It is known that proteolytic digestion of an antibody can lead to the production of antibody fragments. Such smaller fragments of the whole antibody can exhibit antigen binding activity.

**Fv fragments,** consisting only of the V-domains of the heavy and light chains associated with each other may be obtained (Fig. 1B₂). These Fv fragments are monovalent for antigen binding. Smaller fragments such as individual V-domains (domain antibodies or dABs, Ward et al Nature, 341, 544 (1989) and individual CDR's (Williams et al. (1989), Proc. Natl. Acad. Sci, USA. 86: 5537-5541) have also been shown to retain the binding characteristics of the parent antibody although generally most naturally occurring antibodies need both a VH and VL to retain full immunoreactivity.

**Single chain variable fragment (scFv)** constructs comprise a VH and a VL domain of an antibody contained in a single polypeptide chain wherein the domains are separated by a flexible linker of sufficient length (more than 12 amino acids), that forces intramolecular interaction, allowing self-assembly of the two domains into a functional epitope binding site (Bird et al. (1988), Science 242: 423-426) (Fig. 1B₃). These small proteins (MW -25000 Da) generally retain specificity and affinity for their antigen in a single polypeptide and can provide a convenient building block for larger, antigen-specific molecules.

An advantage of using antibody fragments rather than whole antibodies in diagnosis and therapy lies in their smaller size. They are likely to be less immunogenic than whole antibodies and more able to penetrate tissues. A disadvantage associated with the use of such fragments is that they have only one antigen binding site, leading to reduced avidity.

### Monospecific polyvalent antibody constructs

Tetravalent antibodies called "linear Fab" have been described by Miller et al. (2003), J Immunol. 170: 4854-4861 (Fig. 1B₅). The "linear Fab" is composed of a tandem of the same CH1-VH domain, paired with the identical light chain at each CH1-VH position. These molecules have been developed in order to increase the valency of an antibody to enhance its functional affinity through the avidity effect, but they are monospecific.

### 2. Bispecific antibodies

It has been of interest to produce bispecific antibodies (BsAbs) which combine the antigen-binding sites of two antibodies within a single molecule. Thus, they are able to bind two different antigens simultaneously. Besides applications for diagnostic purposes, they pave the way for new therapeutic applications by redirecting potent effector systems to diseased areas or by increasing neutralizing or stimulating activities of antibodies.

### Bispecific bivalent antibodies

Initial attempts to couple the binding specificities of two whole antibodies against different target antigens for therapeutic purposes utilized chemically fused heteroconjugate molecules (Staerz et al.(1985), Nature 314: 628-631).
Bispecific antibodies have been produced from hybrid hybridomas by heterohybridoma techniques and have demonstrated in vitro properties similar to those observed for heteroconjugates (Milstein & Cuello (1983) Nature 305:537-540).

Despite the promising results obtained using heteroconjugates or bispecific antibodies produced from cell fusions as cited above, several factors made them impractical for large scale therapeutic applications. Such factors include: rapid clearance of large heteroconjugates in vivo, the labor intensive techniques required for generating either type of molecule, the need for extensive purification of heteroconjugates away from homoconjugates or mono-specific antibodies and generally low yields.

Genetic engineering has been used with increasing frequency to design, modify, and produce antibodies or antibody derivatives with a desired set of binding properties and effector functions.

A variety of recombinant methods have been developed for efficient production of BsAbs, both as antibody fragments (Carter et al. (1995), J. Hematotherapy 4: 463-470; Pluckthun et al. (1997) Immunotechology 3: 83-105; Todorovska et al. (2001) J. Immunol. Methods 248: 47-66) and full length IgG formats (Carter (2001) J. Immunol. Methods 248: 7-15).

Combining two different scFvs results in BsAb formats with minimal molecular mass, termed **sc-BsAbs or Ta-scFvs** (Mack et al.(1995), Proc. Acad. Sci.USA. 92: 7021-7025; Mallender et al. (1994) J. Biol. Chem. 269: 199-206) (Fig. 1C₁). BsAbs have been constructed by genetically fusing two scFvs via a dimerization functionality such as a leucine zipper (Kostelny et al. (1992) J. Immunol. 148: 1547-53; de Kruif et al. (1996) J. Biol. Chem. 271: 7630-4) (Fig. 1C₂).

**Diabodies** are small bivalent and bispecific antibody fragments (Fig. 1C₃). The fragments comprise a VH connected to a VL on the same polypeptide chain, by using a linker that is too short (less than 12 amino acids) to allow pairing between the two domains on the same chain. The domains are forced to pair intermolecularly with the complementary domains of another chain and create two antigen-binding sites. These dimeric antibody fragments, or "diabodies," are bivalent and bispecific. (Holliger et al. (1993), Proc. Natl. Acad. Sci. USA. 90: 6444-6448). Diabodies are similar in size to a Fab fragment. Polypeptide chains of VH and VL domains joined with linker between 3 and 12 amino acids form predominantly dimers (diabodies), whereas with linker between 0 and 2 amino acid residues, trimers (triabodies) and tetramers (tetrabodies) find favor. In addition to the linker length, the exact pattern of oligomerization seems to depend on the composition as well as the orientation of the V-domains (Hudson et al. (1999), J Immunol Methods 231: 177-189). The predictability of the final structure of diabody molecules is very poor.

Although sc-BsAbs and diabodies based constructs display interesting clinical potential, it was shown that such non-covalently associated molecules are not sufficient stable under physiological conditions. The overall stability of a scFv fragment depends on the intrinsic stability of the VL and VH domains as well as on the stability of the domain interface. Insufficient stability of the VH-VL interface of scFv fragments has often been suggested as a main cause of irreversible scFv inactivation, since transient opening of the interface, which would be allowed by the peptide linker, exposes hydrophobic patches that favor aggregation and therefore instability and poor production yield (Wörn and Plückthun (2001), J. Mol. Biol. 305: 989-1010).

An alternative method of manufacturing bispecific bivalent antigen-binding proteins from VH and VL domains is disclosed in US 5,989,830. Such double head antibody fragments (Fig. 1C₄) are obtained by expressing a dicistronic vector which encodes two polypeptide chains, whereby one polypeptide chain has two times a VH in series by a peptide linker (VH1-linker-VH2) and the other polypeptide chain consisting of complementary VL domains connected in series by a peptide linker (VL1-linker-VL2). Molecular modeling of the constructs suggested the linker size to be long enough to span 30-40 A (15 - 20 amino acids).

However, it was shown that such constructs are not sufficient stable under physiological conditions and that the pairing of the VH and VL polypeptides is not always correct, which leads aggregates with high molecular masses.

### Bispecific polyvalent antibodies

Increasing the valency of an antibody is of interest as it enhances the functional affinity of that antibody due to the avidity effect.
**Polyvalent protein complexes (PPC)** with an increased valency are described in US 2005/0003403 A1. PPCs comprise two polypeptide chains generally arranged laterally to one another (Fig. 1C₅). Each polypeptide chain typically comprises 3 or 4 "v-regions", which comprise amino acid sequences capable of forming an antigen binding site when matched with a corresponding v-region on the opposite polypeptide chain. Up to about 6 "v-regions" can be used on each polypeptide chain. The v-regions of each polypeptide chain are connected linearly to one another and may be connected by interspersed linking regions. When arranged in the form of the PPC, the v-regions on each polypeptide chain form individual antigen binding sites. The complex may contain one or several binding specificities.

However, it was shown that these constructs have the same limits as the ones disclosed in US 5,989,830 namely insufficient phsiological stability and a tendency for aggregation coupled with poor expression yields.

Another method of manufacturing bispecific polyvalent antibodies is disclosed in Meng et al., Clin. Cancer Res., 2004, 10:1274-1281. Such Fc-tagged tandem single chain Fv (Ta-**scFv-Fc) molecules** are tetravalent constructs and are made by the association of two identical chains, each chain comprising a scFv tandem linked to a Fc domain (Fig. 1C₆).
Such constructs derive from Fc-tagged single chain Fv (scFv-Fc) molecules which are made from the association of a scFv to a Fc domain (Fig. 1B₄).

However, the use of such molecules showed aggregation, unstability and poor expression yield (Wu et al. (2001) Prot. Eng. 14: 1025-1033). These are typical stability problems that may occur expressing single chain based antibodies. (Wörn and Plückthun (2001), J. Mol. Biol. 305: 989-1010).

Thus, it is the object of the present invention to provide a bispecific polyvalent antibody by means of which the formation of aggregates can be avoided. Furthermore, it shall have a stability which makes it usable for therapeutic uses.

According to the invention, this is achieved by the subject matters defined in the claims.

### SUMMARY OF THE INVENTION

One subject matter of the present invention is directed to a polyvalent antibody comprising: at least one first polypeptide which is a constant heavy chain and at least one second polypeptide which is a constant light chain, wherein the first polypeptide is attached to two or more variable domains, and the second polypeptide is attached to two or more variable domains.

Another subject matter of the present invention is directed to a dimeric polyvalent antibody comprising two identical polypeptide monomers, wherein each polypeptide monomer comprises: a first polypeptide which is a constant heavy chain and a second polypeptide which is a constant light chain, wherein the first polypeptide is attached to two or more variable domains, and the second polypeptide is attached to two or more variable domains.

In further embodiments, the invention consists of a polyvalent antibody comprising three, four, five, six, seven, eight, nine, ten or more polypeptide monomers, wherein each polypeptide monomer comprises: a first polypeptide which is a constant heavy chain and a second polypeptide which is a constant light chain, wherein the first polypeptide is attached to two or more variable domains, and the second polypeptide is attached to two or more variable domains.

In a preferred embodiment, the two or more variable domains attached to the first polypeptide are variable domains of a heavy and/or light chain, and the two or more variable domains attached to the second polypeptide are variable domains of a heavy and/or light chain.

In a particularly preferred embodiment, the two or more variable domains attached to the first polypeptide are variable domains of a heavy chain, and the two or more variable domains attached to the second polypeptide are variable domains of a light chain.

In general, the two or more variable domains attached to the first polypeptide associate with the two or more variable domains attached to the second polypeptide to form two or more antigen binding sites. The two or more antigen binding sites may be equal or different from each other, the polyvalent antibody being monospecific if the two or more antigen binding sites are all equal, bispecific if the two or more antigen binding sites have two different specificities, and multispecific if the two or more antigen binding sites have three or more different specificities.

The polyvalent antibodies according to the present invention may bind to two or more antigens. In a preferred embodiment, it binds to four antigens, the antibody being tetravalent. The polyvalent antibodies according to the invention may allow a binding efficiency of at least 50%, 60%, 70%, 80% or 90% for the antigen.

In a preferred embodiment of the present invention, the two or more variable domains are linked to each other by one or more linkers. The linkers may be the same or different from each other.

In general, the constant heavy chain is selected from one of the following immunoglobulin classes: γ, α*,*µ*,* ε and δ, in a preferred embodiment, it is a γ constant heavy chain; and the constant light chain is selected from one of the following classes: *k* and λ.
The two or more variable domains and/or the first and second polypeptides may derive from a monoclonal or a polyclonal antibody. Furthermore, the polyvalent antibody according to the present invention may be murine, chimeric, synthetic, humanized or human.

Advantageously, the amino acid sequence of the first polypeptide attached to two or more variable domains is selected from one of the following sequences: SEQ ID NO: 2 and SEQ ID NO: 6, and the amino acid sequence of the second polypeptide attached to two or more variable domains is selected from one of the following sequences: SEQ ID NO: 4 and SEQ ID NO: 8.
The first polypeptide attached to two or more variable domains may be encoded by a DNA sequence which is cloned into one of the following deposited plasmids: DSM19744 and DSM19746, and the second polypeptide attached to two or more variable domains may be encoded by a DNA sequence which is cloned into one of the following deposited plasmids DSM19745 and DSM19747.

Another subject matter of the present invention is directed to a nucleic acid molecule encoding the first polypeptide attached to two or more variable domains of the polyvalent antibodies of the present invention. Said nucleic acid molecule may be DNA or RNA. Advantageously, said DNA may have a nucleic acid sequence selected from one of the following sequences: SEQ ID NO: 1 and SEQ ID NO: 5. The sequence of said DNA may be cloned into one of the following deposited plasmids: DSM19744 and DSM19746. The plasmid containing the DNA molecule encoding the first polypeptide attached to two or more variable domains of the polyvalent antibody may have a nucleic acid sequence selected from one of the following sequences: SEQ ID NO 9 and SEQ ID NO 11.

Another subject matter of the present invention is directed to a nucleic acid molecule encoding the second polypeptide attached to two or more variable domains of the polyvalent antibodies of the present invention. Said nucleic acid molecule may be DNA or RNA. Advantageously, said DNA may have a nucleic acid sequence selected from one of the following sequences: SEQ ID NO: 3 and SEQ ID NO: 7. The sequence of said DNA may be cloned into one of the following deposited plasmids: DSM19745 and DSM19747. The plasmid containing the DNA molecule encoding the second polypeptide attached to two or more variable domains of the polyvalent antibody may have a nucleic acid sequence selected from one of the following sequences: SEQ ID NO 10 and SEQ ID NO 12.

A further subject matter of the present invention refers to a vector comprising said DNA molecules and to a host cell comprising said DNA molecules or said vectors. Preferred host cells are selected from but are not restricted to the group consisting of: HEK293, CHO, NS-0 and COS-7.

The invention further pertains to a process for producing the polyvalent antibodies according to the present invention comprising: transforming an eukaryotic host cell by incorporating a DNA molecule encoding the first polypeptide attached to two or more variable domains of the polyvalent antibody and a DNA molecule encoding the second polypeptide attached to two or more variable domains of the polyvalent antibody; cultivating the host cell under suitable conditions so that said nucleic acid molecules are expressed; causing or allowing said first and second polypeptides to combine to form the polyvalent antibody; and purifying the polyvalent antibody from the culture medium.

Preferred host cells used in said process may be selected from but not restricted to the group consisting of: HEK293, CHO, NS-0 and COS-7.

Another subject matter of the present invention is directed to a pharmaceutical composition comprising an antibody according to the invention and a pharmaceutically acceptable excipient. Said pharmaceutical composition may be used in therapy, for the treatment of, among others, cancer, inflammation, autoimmune diseases, infections, cardiovascular diseases, respiratory diseases, neurological diseases or metabolic diseases. Said pharmaceutical composition may also be used in diagnostic, to diagnose said diseases.

The present invention further refers to the use of an antibody according to the invention in the manufacture of a medicament for the therapeutic and/or prophylactic treatment of, among others, cancer, inflammation, autoimmune diseases, infections, cardiovascular diseases, respiratory diseases, neurological diseases or metabolic diseases. It also refers to the use of an antibody according to the invention in the manufacture of a diagnostic composition to diagnose, among others, cancer, inflammation, autoimmune diseases, infections, cardiovascular diseases, respiratory diseases, neurological diseases or metabolic diseases.

Another subject of the present invention is directed to an assay for an antigen using a polyvalent antibody according to the invention, comprising: incubating the polyvalent antibody and an antigen under conditions which permit binding of the antigen to the polyvalent antibody; and detecting the antigen-polyvalent antibody binding.

### DETAILED DESCRIPTION OF THE INVENTION

It is the object of the present invention to provide a bispecific polyvalent antibody which is stable and does not aggregate. Therefore, the subject matter of the present invention relates to a polyvalent antibody construct which has a superior stability. Such a construct is suitable for diagnostic and therapeutic purposes.

The present invention is based on the applicant's insights that the stability of a polyvalent antibody can be increased by creating a bispecific molecule resembling and keeping the stability characteristics of a natural antibody, expressing the heavy and light chains on two different polypeptides and incorporating the heavy and light constant regions in order to stabilize the VH/VL interaction and to force the correct pairing of the cognate VH and VL domains.

According to the invention, the applicant's insights are used to provide a dimeric polyvalent antibody construct wherein a monomer comprises a heavy chain consisting of two or more variable heavy chain domains (VH1, VH2, etc...) deriving from the same or different antibodies, attached to each other by a linker and fused to an antibody constant region; and a light chain consisting of two or more variable light chain domains (VL1, VL2, etc...) deriving from the same or different antibodies, attached to each other by a linker and fused to a constant light region.

Thus, the resulting construct is a natural antibody construct which is elongated at its N-terminal regions on the corresponding heavy and light chains, respectively by one or more variable heavy domains and one or more variable light domains.

A preferred embodiment of the present invention refers to a tetravalent bispecific tandem immunoglobulin (TBTI), which comprises two identical tandems of VH/VL pairs, each having two antibody binding sites, preferably non-identical. One of the TBTI chains includes an immunoglobulin CH1 and hinge region and at least one heavy chain constant domain that is capable of self-association to form a dimer (e.g., a CH2 and/or a CR3 domain). The second chain includes a constant light domain, allowing the correct pairing of the heavy and light chains through the strong interaction between CH1/CL and conferring stability to the TBTI molecule. This novel TBTI format offer several advantages as compared to previously described bispecific antibody formats. TBTIs can be produced that are bispecific and bivalent. TBTI have no constraints regarding selection of antigen-binding sites, and Fc constant domains and associated functions can be retained. The TBTI molecules are substantially homogeneous and can be produced in mammalian, or other cells without further processing. Notably, because the binding characteristics of bispecific TBTIs for each of two different antigens are comparable to those of natural antibodies for each antigen, a single pharmaceutical TBTI preparation can be used in place of a combination of two monospecific antibodies. The TBTIs can also be monospecific (i.e., four identical antibody binding sites). Further, the TBTI of the invention are efficiently produced in mammalian cells, and can be purified by a conventional single step protein A chromatography.

An additional advantage of the present invention, for the association of the bispecific polyvalent construct over existing techniques, lies in the given nature of the fused domains utilized for this purpose. One of the major disadvantages of chemical modification or other approaches (i.e. the Jun/Fos based dimerization, Fig. 1C₂) concerns their potential to elicit an immune response against "foreign" chemical constituents and peptide sequences when used in human therapy. TBTIs of the invention comprise at least one IgG constant domain that is capable of self association, such as, for example, CH2 or CH3 of IgG, IgA, or IgD, or CH2, CH3, or CR4 of IgE or IgM. Accordingly, TBTIs possess the immunological functions associated with such domains that are present, such as Fc receptor binding, complement dependent cytotoxicity (CDC), or antibody dependent cell-mediated cytoxicity (ADCC). The usage of an intact antibody molecule to achieve bispecificity of the described engineered TBTI antibody avoids this problem. The natural antibody domains used in the present invention are not expected to induce an immune response in humans.

### Definitions:

The term "antibody" as used in the present invention refers to a full-length immunoglobulin molecule. The term "immunoglobulin" is used interchangeably with "antibody" herein. By "full-length immunoglobulin" herein is meant the structure that constitutes the natural biological form of an antibody, including variable and constant regions. It refers to a monomer comprising one antibody heavy chain (abbreviated herein as HC) and one antibody light chain (abbreviated herein as LC); or to a dimer comprising two antibody heavy chains and two antibody light chains, wherein the heavy chains are inter-connected by disulfide bonds. It further refers to a multimer comprising three, four, five, six, seven, eight, nine, ten or more antibody heavy chains and three, four, five, six, seven, eight, nine, ten or more antibody light chains, wherein the heavy chains are inter-connected by disulfide bonds.

The term "heavy chain" as used in the present invention refers to an antibody heavy chain which is comprised of two or more heavy chain variable domains (abbreviated herein as VH) and a heavy chain constant region (abbreviated herein as CH). The heavy chain constant region may be comprised of one domain, two domains, three domains or four domains. In a preferred embodiment, the heavy chain constant region is comprised of four domains: CH1, CH2 and CH3, and a hinge region between CH1 and CH2. Heavy chains are classified as gamma (γ), alpha (α), mu (µ), epsilon (ε) or delta (δ), and define the antibody's isotype as IgG, IgA, IgM, IgE, and IgD, respectively.

The term "light chain" as used in the present invention refers to an antibody light chain which is comprised of two or more light chain variable domains (abbreviated herein as VL) and a light chain constant region (abbreviated herein as CL). The light chain constant region is comprised of one domain, CL. Human light chains are classified as kappa (*k*) and lamda (λ) light chains.

The variable domains VH and VL can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4.

A variable domain of the heavy chain associates with a variable domain of the light chain to form a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "hinge" or "hinge region" as used in the present invention refers to the flexible polypeptide comprising the amino acids between the first and second constant domains of an antibody.

The subunit structures and three-dimensional configurations of different classes of antibodies are well known (Cellular and Molecular Immunology; Abul K. Abbas, Andrew Lichtman; 2005).

The term "polypeptide" as used in the present invention refers to a polymer of amino acid residues. The term applies to amino acid polymers in which one or more amino acid residue is an artificial chemical mimetic of a corresponding naturally occurring amino acid, as well as to naturally occurring amino acid polymers and non-naturally occurring amino acid polymer.

The term "first polypeptide" as used in the present invention refers to a polypeptide which is to be associated with a second polypeptide. The first and second polypeptides are associated through a disulfide bond. The first polypeptide may comprise one, two or three antibody heavy constant domains. In a preferred embodiment, it comprises three antibody heavy constant domains: CH1, CH2 and CH3, and a hinge region between CH1 and CH2. Said heavy constant domains may derive from an antibody which is murine, chimeric, synthetic, humanized or human, and monoclonal or polyclonal. The first polypeptide is attached to two or more variable domains, preferably variable domains of an antibody heavy chain.

The term "second polypeptide" as used in the present invention refers to a polypeptide which is to be associated with the first polypeptide through a disulfide bond. The second polypeptide comprises an antibody light constant region CL. Said light constant region may derive from an antibody which is murine, chimeric, synthetic, humanized or human, and monoclonal or polyclonal. The second polypeptide is attached to two or more variable domains, preferably variable domains of an antibody light chain.

The term "polyvalent antibody" as used in the present invention refers to an antibody comprising two or more antigen binding sites, thus being able to bind two or more antigens, which may have the same or a different structure, simultaneously. The term "bivalent" means that the antibody comprises two antigen binding sites. The term "tetravalent" means that the antibody comprises four antigen binding sites.

The term "antigen binding site" as used in the present invention refers to the part of the antibody which comprises the area which specifically binds to and is complementary to part or all of an antigen. Where an antigen is large, an antibody may only bind to a particular part of the antigen, which part is termed on epitope. An antigen binding domain may be provided by one or more antibody variable domains. Preferably, an antigen binding domain is made of the association of an antibody light chain variable domain (VL) and an antibody heavy chain variable domain (VH).

The term "antigen" as used in the present invention refers to a molecule or a portion of a molecule capable of being bound by the antibodies of the present invention. An antigen can have one or more than one epitope. Examples of antigens recognized by the antibodies of the present invention include, but are not limited to, serum proteins, e.g. cytokines such as IL4, IL5, IL9 and IL13, bioactive peptides, cell surface molecules, e.g. receptors, transporters, ion-channels, viral and bacterial proteins, RAGE (Receptor for Advanced Glycosylation End Products), GPVI and collagen.

The term "monospecific" as used in the present invention means that the polyvalent antibody of the present invention recognizes only one antigen, all the antigen binding sites being identical.

The tem "bispecific" as used in the present invention means that the polyvalent antibody of the present invention recognizes two different epitopes on the same or on two different antigens.

The term "multispecific" as used in the present invention means that the polyvalent antibody of the present invention recognizes multiple different epitopes on the same or on multiple different antigens.

The term "chimeric antibody" as used in the present invention refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (e.g.: U.S. Pat. No. 4,816,567; Morrison et al., Proc. Nat. Acad. Sci. USA, 81:6851-6855 (1984)).

The term "humanized antibody" as used in the present invention refers to a recombinant antibody in which the CDRs from an antibody from one species, e.g. a rodent antibody, are transferred from the heavy and light variable chains of the rodent antibody into heavy and light variable domains of human origin. The constant domains of the antibody molecule are derived from those of a human antibody.

Methods for making chimeric and humanized antibodies are also known in the art. For example, methods for making chimeric antibodies include those described in patents by Boss (Celltech) and by Cabilly (Genentech) (U.S. Pat. No. 4,816,397; U.S. Pat. No. 4,816,567).

The term "human antibody" as used in the present invention refers to an antibody obtained from transgenic mice that have been "engineered" to produce specific human antibodies in response to antigenic challenge. In this technique, elements of the human heavy and light chain loci are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994). A fully human antibody also can be constructed by genetic or chromosomal transfection methods, as well as phage display technology, all of which are known in the art. See for example, McCafferty et al., Nature 348:552-553 (1990) for the production of human antibodies and fragments thereof in vitro, from immunoglobulin variable domain gene repertoires from unimmunized donors. In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see e.g. Johnson and Chiswell, Current Opinion in Structural Biology 3:5564-571 (1993).

The term "monoclonal antibody" as used in the present invention refers to an antibody obtained from a population of substantially homogeneous antibodies, i.e., the individual antibodies comprising the population are identical except for possible naturally occurring modifications that may be present in minor amounts. Monoclonal antibodies are highly specific, being directed against a single antigenic site (epitope).. In addition to their specificity, the monoclonal antibodies are advantageous in that they are synthesized by hybridoma cell culture, uncontaminated by other immunoglobulins. Monoclonal antibodies may be obtained by methods known to those skilled in the art. See, for example Kohler and Milstein, Nature 256:495 497 (1975); U.S. Pat. No. 4,376,110; Ausubel et al, eds., CURRENT PROTOCOLS IN MOLECULAR BIOLOGY, Greene Publishing Assoc. and Wiley Interscience, N.Y., (1987, 1992); and Harlow and Lane ANTIBODIES: A LABORATORY MANUAL Cold Spring Harbor Laboratory (1988).

The term "polyclonal antibody" as used in the present invention refers to a mixture of different antibody molecules each reacting possibly with a different immunogenic determinant (epitope) of an antigen.
It encompasses a polyclonal antibody isolated or purified from mammalian blood, secretions, or other fluids, or from eggs, as well as a mixture of different monoclonal antibodies, and finally a polyclonal antibody may be produced as a recombinant polyclonal antibody.

The term "linker" as used in the present invention refers to a peptide adapted to connect the variable domains of the antibody constructs of the present invention.. The peptide linker may contain any amino acids, the amino acids glycine (G) and serine (S) being preferred. The linkers may be equal or differ from each other between and within the heavy chain polypeptide and the light chain polypeptide. Furthermore, the linker may have a length of 1, 2, 3, 4, 5, 6, 7, 8, 9, 10, 11, 12, 13, 14, 15, 16, 17, 18, 19 or 20 amino acids. A preferred peptide linker unit for the heavy chain domains as for the light chain domains is GGGS. The number of linker units of the heavy chain and of the light chain may be equal (symmetrical order) or differ from each other (asymmetrical order).

A peptide linker is preferably long enough to provide an adequate degree of flexibility to prevent the antibody moieties from interfering with each others activity, for example by steric hindrance, to allow for proper protein folding and, if necessary, to allow the antibody molecules to interact with two or more, possibly widely spaced, receptors on the same cell; yet it is preferably short enough to allow the antibody moieties to remain stable in the cell. Therefore, the length, composition and/or conformation of the peptide linkers can readily be selected by one skilled in the art in order to optimize the desired properties of the polyvalent antibody.

The term "nucleic acid molecule encoding" as used in the present invention refers to a nucleic acid which directs the expression of a specific protein or peptide. The term includes both the DNA molecule that is transcribed into RNA and the RNA molecule that is translated into protein. It includes both full length nucleic acid molecules as well as shorter molecules derived from the full length molecules.

The term "nucleic acid sequence" as used in the present invention refers to a succession of letters representing the primary structure of a real or hypothetical DNA or RNA molecule or strand, with the capacity to carry information. It is understood that a particular nucleic acid sequence includes the degenerate codons of the native sequence or sequences which may be introduced to provide codon preference in a specific host cell. With regard to its biological function, which may depend on context, a sequence may be *sense* or *anti-sense,* and either coding or noncoding.

The term "vector" as used in the present invention comprises cloning vectors and expression vectors.
The term "cloning vector" refers to a DNA molecule, such as a plasmid, cosmid, phagemid, or bacteriophage, which has the capability of replicating autonomously in a host cell and which is used to transform cells for gene manipulation. Cloning vectors typically contain one or a small number of restriction endonuclease recognition sites at which foreign DNA molecules may be inserted in a determinable fashion without loss of an essential biological function of the vector, as well as a marker gene which is suitable for use in the identification and selection of cells transformed with the cloning vector. Marker genes typically include genes that provide tetracycline resistance or ampicillin resistance.

The term "expression vector" refers to a DNA molecule comprising a cloned structural gene encoding a foreign protein which provides the expression of the foreign protein in a recombinant host. Typically, the expression of the cloned gene is placed under the control of (i.e., operably linked to) certain regulatory sequences such as promoter and enhancer sequences. Promoter sequences may be either constitutive or inducible.

The term "host cell" as used in the present invention refers to a prokaryotic or eukaryotic cell which contains either a cloning vector or expression vector. This term is also meant to include those prokaryotic or eukaryotic cells that have been genetically engineered to contain the cloned gene(s) in the chromosome or genome of the host cell. The host cell is not limited to a unicellular organism such as E. coli and yeast. Cells from multicellular organisms such as mammals, insects, and plants are also contemplated as host cells in the context of this invention. For examples of suitable hosts, see Sambrook et al., MOLECULAR CLONING: A LABORATORY MANUAL, Second Edition, Cold Spring Harbor Laboratory, Cold Spring Harbor, N.Y. (1989). A mammalian host cell may be of any mammalian origin and include, at least cells of human, bovine, canine, murine, rattus, equine, porcine, feline and non-human primate origin.
Preferred host cells are eukaryotic host cells such as HEK293 and derivatives, CHO and derivatives, NS-0, COS-7 and derivatives.

Techniques for synthesising genes, incorporating them into hosts and expressing genes in hosts are well known in the art and the skilled person would readily be able to put the invention into effect using common general knowledge (Current protocols in molecular biology, John Wiley & Sons Inc, ISBN: 0471250961). Proteins according to the invention may be recovered and purified using conventional techniques such as affinity chromatography, ion exchange chromatography or gel filtration chromatography.

The polyvalent antibodies of the invention may be used among other things for in vivo diagnosis or treatment.

The term "treatment" as used in the present invention refers to both therapeutic treatment and prophylactic or preventative measures. It refers to preventing, curing, reversing, attenuating, alleviating, minimizing, suppressing or halting the deleterious effects of a disease state, disease progression, disease causative agent (e.g., bacteria or viruses) or other abnormal condition.

Thus the invention also includes polyvalent antibodies having attached thereto diagnostically or therapeutically functional effector molecules, atoms or other species. For example, the antibody may have a radioactive diagnostic label or radioactive cytotoxic atom or metal or cytotoxic species, e.g. ricin chain, attached thereto for in vivo diagnosis or therapy of cancer.

Moreover, the antibodies according to the invention may be used in immunoassays, in purification methods and in other methods in which immunoglobulins or fragments thereof are used. Such uses are well-known in the art.

Accordingly, the invention also provides compositions comprising the polyvalent antibodies according to the invention, conveniently in combination with a pharmaceutically acceptable carrier, diluent or excipient which are conventional in the art.

The term "pharmaceutical composition" as used in the present invention refers to formulations of various preparations. The formulations containing therapeutically effective amounts of the polyvalent antibodies are either sterile liquid solutions, liquid suspensions or lyophilized versions and optionally contain stabilizers or excipients.

The term "disorder" as used in the present invention refers to any condition that would benefit from treatment with the antibody of the present invention. This includes chronic and acute disorders or diseases including those pathological conditions which predispose the mammal to the disorder in question. Non-limiting examples of disorders to be treated herein include cancers, inflammation, autoimmune diseases, infections, cardiovascular diseases, respiratory diseases, neurological diseases and metabolic diseases.

The term "cancer" as used in the present invention refers to or describes the physiological condition in mammals that is typically characterized by unregulated cell growth. Examples of cancer include but are not limited to, carcinoma, lymphoma, blastoma, sarcoma, and leukemia.

The term "autoimmune disease" as used in the present invention refers to a non-malignant disease or disorder arising from and directed against an individual's own tissues. Examples of autoimmune diseases or disorders include, but are not limited to, inflammatory responses such as inflammatory skin diseases including psoriasis and dermatitis; allergic conditions such as eczema and asthma; other conditions involving infiltration of T cells and chronic inflammatory responses; atherosclerosis; diabetes mellitus (e. g. Type I diabetes mellitus or insulin dependent diabetes mellitis); multiple sclerosis and central nervous system (CNS) inflammatory disorder.

The invention is explained by the below examples. The following preparations and examples are given to enable those skilled in the art to more clearly understand and to practice the present invention. The present invention, however, is not limited in scope by the exemplified embodiments, which are intended as illustrations of single aspects of the invention only, and the methods which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims.

Techniques for producing and purifying biological subject matter for the purposes of the present invention can be found in Current protocols in molecular biology, John Wiley & Sons Inc, ISBN: 0471250961.

### EXAMPLES

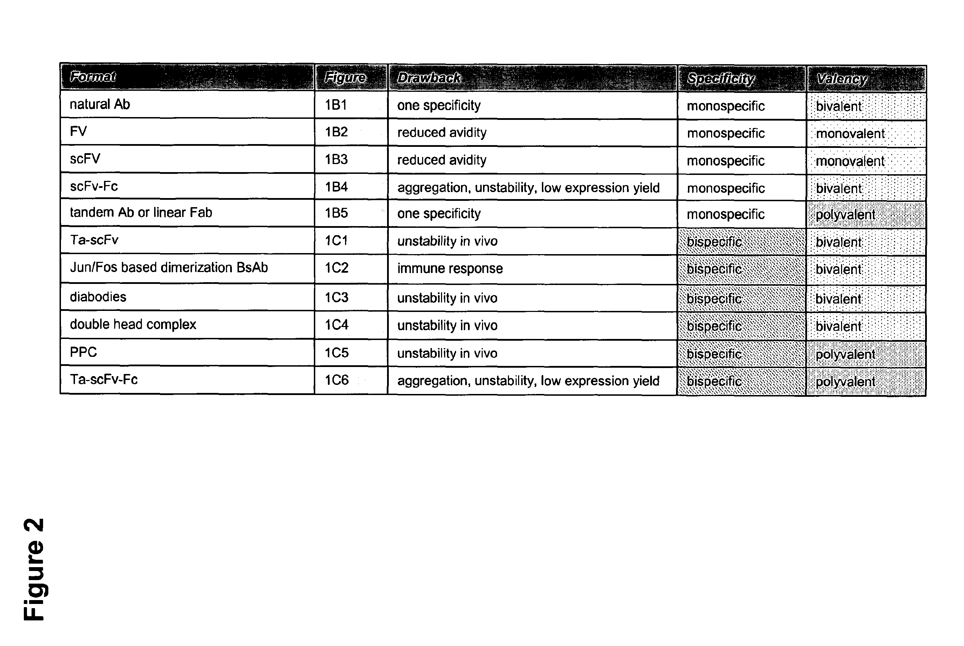

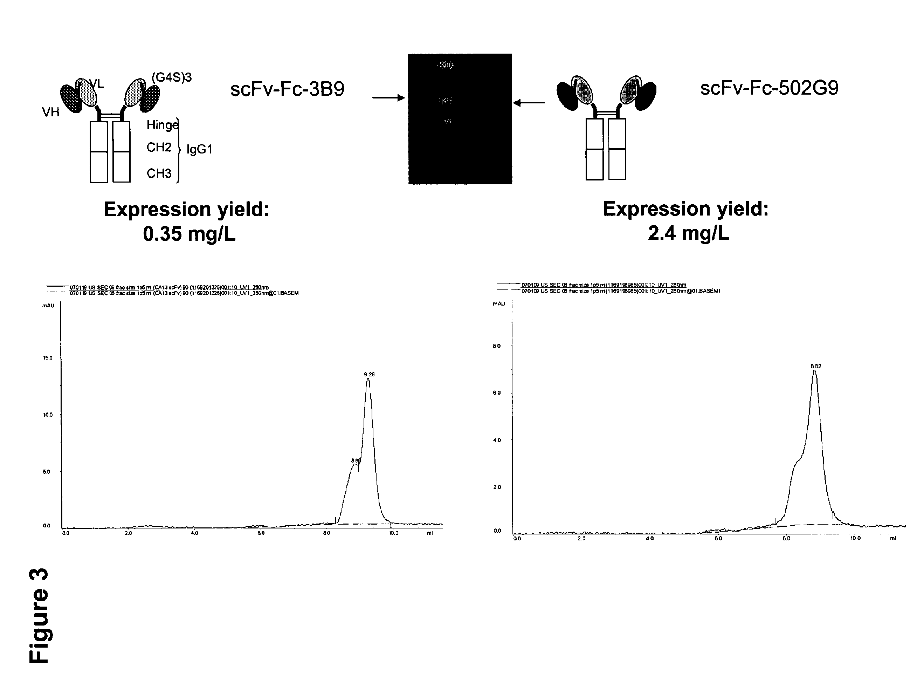

In order to evaluate a selected format for the expression of tetravalent bispecific antibodies, Fc-tagged tandem single chain Fv (Ta-scFv-Fc) molecules (Meng et al., Clin. Cancer Res. (2004), 10: 1274-1281) (Fig. 1B₁₀) based on two published amino acid sequences for anti-IL13 (WO 2005/007699 A2, antibody BAK502G9) and anti-IL4 (US005914110A, antibody 3B9) were constructed.
In a first step, Fc-tagged single chain Fv (scFv-Fc) molecules were constructed. Although both antibodies sequences expressed well as IgG molecules, they showed very low expression and poor stability when the respective variable domains were fused to each other by a (G4S)3 linker to form scFv-Fcs (Fig. 1 B₄, Fig. 2).
In a second step, tetravalent bispecific Fc-tagged tandem single chain Fv (Ta-scFv-Fc) molecules were constructed by fusion of two scFv molecules by a G4S inter-scFv-linker. But their expression failed totally.
Apparently IgGs with excellent biophysical properties, the anti-IL13 antibody BAK502G9 and the anti-IL4 antibody 3B9 are not implicitly suitable to be expressed as scFv molecules. The problems observed were due to aggregation, instability and poor expression yield.
Because of the unpredictable effecty on stability and expression yields due to domain fusion into a specific antibody format, the usage of an intact IgG molecule as backbone and acceptor substrate for an additional Fv domain was investigated (Fig. 3).

### Generation of the variable heavy and light domains of the 3B9 MAb and the 502G9 MAb

Amino acid sequences of the variable heavy and light chains of the anti-IL4 MAb derived from US005914110A (antibody 3B9) were backtranslated into nucleotide sequences building blocks and fused together using a modified protocol of the overlap extension PCR (OE-PCR) described by Young L. and Dong Q. (Nucl. Acids Res. (2004), 32(7), e59). PCR products were cloned into into the pCR^{®}4-TOPO using the Invitrogen TOPO TA cloning kit (Cat #: 45-0641) and sequenced using M13forward and M13 reverse primers.

### Construction of the scFv-Fc and Ta-scFv-Fc molecules

The VH and VL domains were separately PCR-amplified from DNA preparations using primers that anneal in their respective inferred leader peptides (forward primer with a 5' Nhel restriction site) and the variable domains (reverse primer, being tagged at the 5' end with the (G4S)3-linker encoding sequence).

### Construction of TBTI-1

Both sequences for the variable heavy and light domains were generated by PCR with a 5'-BamHl restriction site at their respective 5'-ends encoding part of the (G4S)-linker sequence (GGA-GGC-GGA-GGA-TCC-VH3B9). The 3' sequence of the VH3B9 ended with an Apal restriction site encoding the first amino acids of the CH1 domain. The VH3B9Apal was fused to its constant domain (Apal-IGHG1-HindIII (Q569F4, with deletion of the terminal Lys)) through their Apal restriction sites, creating the heavy chain of the 3B9MAb.
The 3'-end of the VL3B9 ended with a BsiWI restriction site encoding the first amino acids of the Ck domain. The VL3B9BsiWI was fused to its constant domain (BsiWI-IGKC-HindIII (SWISS-PROT Q502W4) through their BsiWI restriction sites, creating the light chain of the 3B9 MAb.
All intermediate PCR fragments were cloned into into the pCR^{®}4-TOPO using the Invitrogen TOPO TA cloning kit (Cat #: 45-0641) and sequenced using M13forward and M13 reverse primers.
To generate the TBTI-1 molecule, the amino acid sequence of the variable heavy and light chains of the anti-IL13 MAb (WO 2005/007699 A2, antibody BAK502G9) were backtranslated into nucleotide sequences and generated using a modified protocol of the overlap extension PCR (OE-PCR). Both sequences were generated with a Nhel restriction site at their respective 5'-ends, followed by an ATG start codon, a leader peptide encoding sequence, the respective VH502G9 or VL502G9, and a common 3'-end encoding the (G4S)-linker (Nhel-Leader-VH502G9- GGA-GGC-GGA-GGA-TCC). The variable heavy chain of the Anti-IL13 MAb 502G9 was fused to the heavy chain of anti IL-4MAb 3B9 through their BamHI restriction sites, creating the heavy chain of TBTI-1.The variable light chain of the anti-IL13 MAb 502G9 was fused to the light chain of anti IL-4MAb 3B9 through their BamHI restriction sites, creating the light chain of TBTI-1 (SEQ#1).

TBTI-1 heavy chain: Nhel- Leader peptide-VH502G9-(G4S) linker- VH3B9-IGHG1- HindIII

TBTI-1 light chain: Nhel- Leader peptide-VL502G9-(G4S) linker- VL3B9-IGKC- Hindlll

The heavy and light chains of TBTI-1 were digested with Nhel and HindIII and each ligated into the Nhel/HindIII sites of the episomal expression vector pXL, an analogon of the pTT vector described by Durocher et al. (2002), Nucl. Acids Res. 30(2), E9, creating the mammalian expression vectors pXL-TBTI-1-HC and pXL-TBTI-1-LC.

### Construction of TBTI-2

Both the heavy and light chain sequences were generated with a 5'-BamHI restriction site at their respective 5'-ends and fused to their constant domains in the same way as describe for example 1. To generate the TBTI-2 molecule, the amino acid sequence of the variable heavy and light chains of the anti-IL4 MAb 3B9 (US005914110A) were backtranslated into nucleotide sequences and produced using OE-PCR. Both sequences were generated in analogy to the described procedure in example 1. The variable heavy chain of the Anti-IL4 MAb 3B9 was fused to the heavy chain of anti IL-13MAb 502G9 through their BamHI restriction sites, creating the heavy chain of TBTI-2. The variable light chain of the anti-IL4 MAb 3B9 was fused to the light chain of anti IL-13MAb 502G9 through their BamHI restriction sites, creating the light chain of TBTI-2 (SEQ#2). Thus the TBTI-2 molecule is composed in a different orientation of the identical antibody domains described in TBTI-1.

TBTI-2 heavy chain: Nhel- Leader peptide-VH3B9-(G4S) linker- VH502G9-IGHG1- Hindlll

TBTI-2 light chain: Nhel- Leader peptide-VL3B9-(G4S) linker- VL502G9-IGKC- Hindlll

The heavy and light chains of TBTI-2 were digested with Nhel and HindIII and each ligated into the Nhel/HindIII sites of the episomal expression vector pXL, creating the mammalian expression vectors pXL-TBTI-2-HC and pXL-TBTI-2-LC.

### Production and Characterization of TBTI molecules

The plasmids pXL-TBTI-HC and pXL-TBTI-LC were propagated in E.coli DH5a. For transfection the plasmids were preparated from E.coli using the Qiagen EndoFree Plasmid Maxi Kit.

For transfection HEK293FreeStyle cells (Invitrogen) were seeded at 3 x 10⁵ cells/mL in 100 mL volume of serum-free FreeStyle medium (Invitrogen) in a 500 mL shaker flask. Cells were cultured in a 37°C incubator with a humidified atmosphere of 8 % CO₂, on an orbital shaker platform rotating at 110 rpm. Three days post-seeding viable and total cell were determined with a CASY electronic cell counter (Schärfe System GmbH). Cells with a viability greater than 90% were used for transfection at a cell density of 1-1.5 x 10⁶ cells/mL. 100 mL cells were transfered in a 225 cm² flask and transfected with a mix of pXL-TBTI-HC and pXL-TBTI-LC (30µg each) using FugeneHD (Roche) at a DNA:FugeneHD ratio of 2:7, at conditions described by the manufacturer. 24 hours posttransfection cells were washed and resuspended in 100mL of serum-free FreeStyle medium, transfered into a 500 mL shaker flask and cultured for 7 days in a 37°C incubator (8 % CO₂) on an orbital shaker platform rotating at 110 rpm.

A Nunc F96-MaxiSorp-immuno plate was coated with goat anti-Human IgG (Fc specific) [NatuTec A80-104A]. The antibody was diluted to 10 ug/ml in carbonate coating buffer (50 mM sodium carbonate pH 9.6) and dispensed at 50 uL per well. Seal the plate with adhesive tape, and store overnight at 4 C. The plate was washed 3 times with Wash buffer (PBS pH 7,4 0,1 % Tween20). 150 uL of Blocking solution (1% BSA / PBS) was dispensed into each well to cover the plate. After 1 hour at RT the plate was washed 3 times with Wash buffer. 100 uL of sample or standards (in a range from 1500 ng/ml to 120 ng/ml) were added and let sit for 1 hours at RT. The plate was washed 3 times with Wash buffer. 100 uL of goat anti-Human IgG-FC - HRP conjugate [NatuTec A80-104P-60] diluted 1:10.000 were add using Incubation solution (0,1%BSA, PBS pH7,4, 0,05% Tween20) After 1 hour incubation at RT, the plate was washed 3 times with Wash buffer. 100 uL of ABTS substrate (10 mg ABTS tablet (Pierce 34026) in ml of 0.1 M Na₂HPO₄, 0.05 M citric acid solution, pH 5,0. Addition of 10 uL of 30% H₂O₂/ 10 ml Subtratbuffer prior to use) were dispensed to each well, allow the color to develop. After the color has developed (approximately 10 to 15 minutes), 50 uL of 1% SDS Solution were added to stop the reaction. The plate were read at A405.

Proteins were purified by affinity chromatography on Protein A (HiTrap™ Protein A HP Columns, GE Life Sciences) after elution from the column with 25 mM citrate pH 3,5 buffer. The monoclonal antibodies were formulated in PBS and 0.22 µm filtered. Protein concentration was determined by measurement of absorbance at 280 nm. Each batch was analyzed using a Protein 200 Plus LabChip kit on the Agilent 2100 bioanalyzer under reducing and non-reducing conditions to determine the purity and the molecular weight of each subunit and of the monomer. Each protein lot was also analyzed by analytical gel filtration (TSKgel G3000SWXL, Tosoh Bioscience; isocratic flow rate of 1 ml/min in 100 mM sodium phosphate buffer pH 6.7 containing 100 mM sodium sulfate) to determine the homogeneity of the preparation and to quantify aggregate levels. Biacore analysis (Biacore 3000, GE Life Sciences) was used to determine binding affinities of the purified protein complexes. For this purpose a sandwich assay using a first, species specific antibody (MAB 1302, Chemicon) for capture and orientation of the investigated antibodies was used. The capture antibody was immobilied via primary amine groups on a CM5 chip (GE Life Sciences) using standard procedures. Binding kinetics were measured after binding of the antibody under investigation with the relevant antigenes (e.g. recombinant human IL-4 and IL-13, Chemicon) over a concentration range between 0 to 50 nM in HBS EP running buffer. Binding kinetics were calculated in the Biacore evaluation program package. To investigate additive binding of both antigens, a wizard-driven co-inject method has been applied in which one antigen was injected immediately followed by the antigen mix. Those skilled in the art will recognize, or be able to ascertain using no more than routine experimentation, many equivalents to the specific embodiments of the invention described herein. Such equivalents are intended to be encompassed by the following claims.

Protein stability was analyzed by incubating the TBTI-molecule in PBS at 37°C at a concentration of 0,5 mg/ml for at least 10 days. Aggregation, degradation and drop of affinity was determined by size exclusion chromatography, Protein 200 Plus LabChip kit and Biacore analysis respectively.

### Results

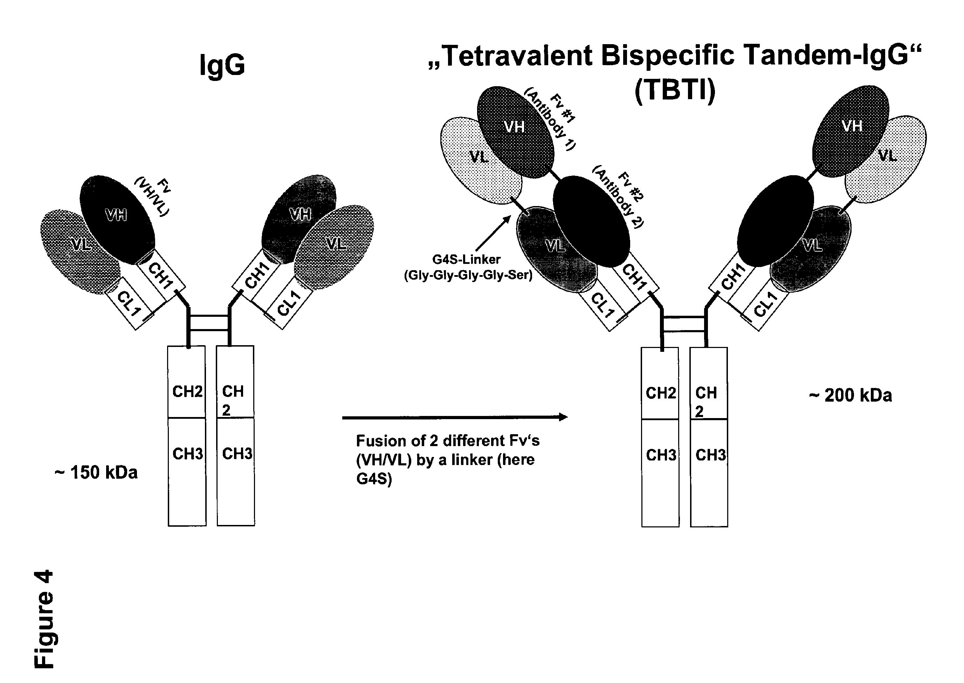

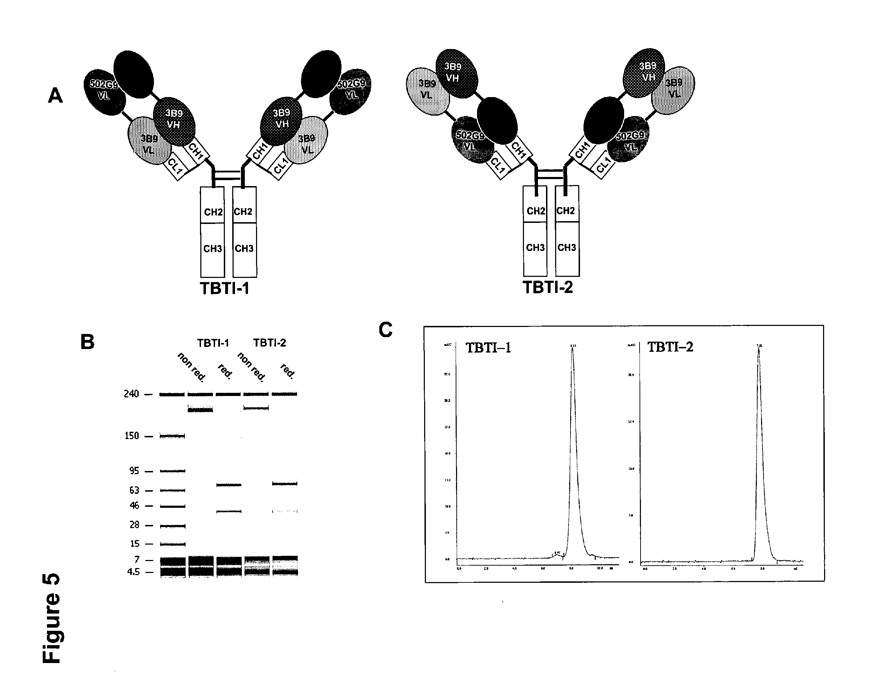

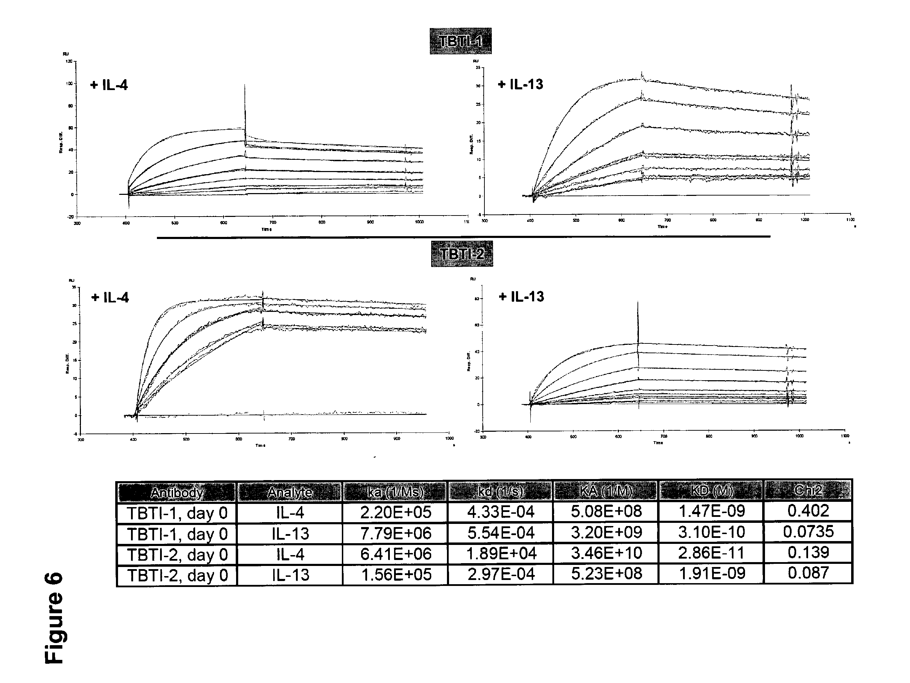

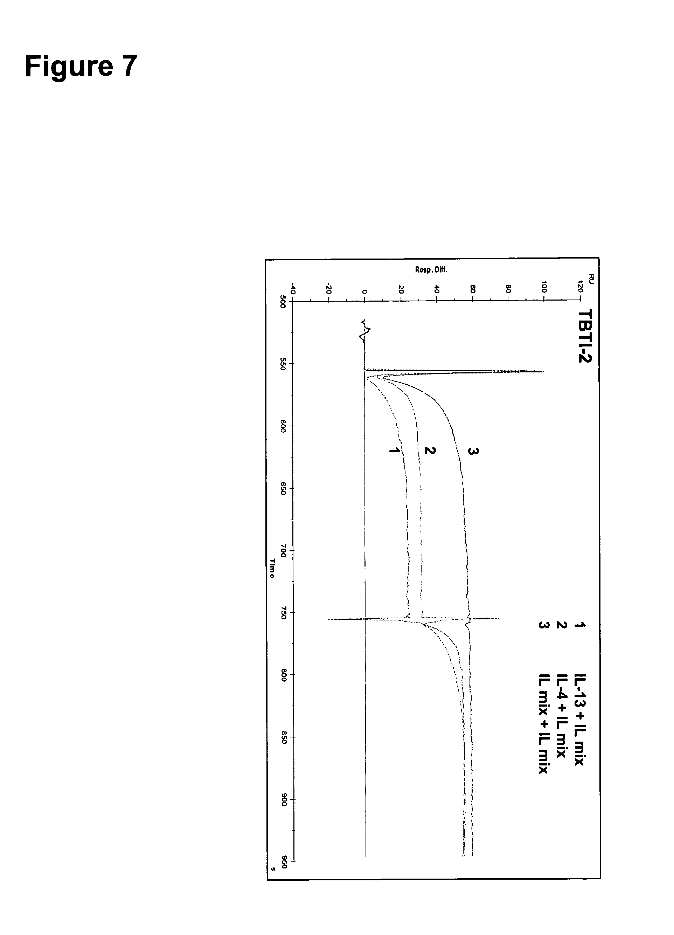

In the first construct called TBTI-1, the anti-IL13 domain is located at theN-terminus and the anti-IL4 domain in the second domain in the TBTI molecule. In a second construct (TBTI-2) the order of the variable domains of TBTI-1 was switched (Fig. 4A). The Agilent chip profiles (reducing and non reducing conditions) of the corresponding TBTI-1 and 2 protein complexes show bands of expected apparent molecular weights: 200kDa for non reduced probes and -70 kDa and -40 kDa for the reduced probes. The TBTI molecules are not degraded (Fig. 4B). The size exclusion profile of TBTI-2 showed no high molecular weight aggregates, whereas TBTI-1 shows minor amounts of aggregates (-1.2 %), indicating a negligible tendency to aggregation in that particular orientation of the Fvs (Fig. 4C). The affinity of TBTI molecules to each antigen is dependent on the position of the variable region fragments (Fv) (Fig. 5). For the TBTI-1 molecule the dissociation kinetic (KD) for IL13 is with 3,101x10⁻¹⁰ M lower as in the TBTI-2 molecule (1,91x10⁻⁹ M), whereas the KD for IL4 is with 1,47x10⁻⁹ M higher in TBTI-1 than in TBTI-2 (2,86x10⁻¹¹ M). This difference is mainly due to different on rates (ka) as KD is the quotient of kd/ka. Thus, for one antigen, the ka is always higher whenever the Fv is located in the N-terminal part of the TBTI molecule.
The TBTI molecule is able to bind both Interleukins consecutively and simultaneously in an additive manner as is shown exemplary for TBTI-2 (Fig. 6). The binding of one antigen does not interfere with the binding of the second antigen. The binding efficiency of both antigen binding domains is close to 100% for each interleukin.
The TBTI molecule is stable after incubation in phosphate buffer saline (PBS) over 5 days at 37°C. The molecule shows neither degradation, as demonstrated by SDS-PAGE (Fig. 7A) nor aggregation as shown by SEC analysis (Fig. 7B) even after 12 days at 37°C. Its ability to bind both antigens with known kinetic is also not affected by the 37°C incubation (Fig. 7C).

### DESCRIPTION OF THE FIGURES

Figure 1: Panel showing different recombinant antibody structures,
   A. polyvalent antibody, e.g. bispecific tetravalent antibody, according to the invention,
   B. different structures of monospecific antibodies from known prior art,
   C. different structures of bispecific antibodies from known prior art.
Figure 2: Table summarizing the characteristics of the different antibody structures from known prior art.
Figure 3: Expression of 3B9 and 502G9 as Fc tagged scFv.
Figure 4: Panel showing a drawing of the differences between an IgG and the TBTI molecule.
Figure 5: Graphical representation of TBTI-1 and TBTI-2 molecules with the different FV orientation (A), an Agilent2100 bioanalyzer gel-like image showing the molecular weight of the TBTI molecules under reducing and non reducing conditions (B) and a size exclusion chromatography profile of TBTI-1 and TBTI-2 (C).
Figure 6: Sensograms and affinity data for the binding of IL-4 and IL-13 to TBTI-1 and TBTI-2.
Figure 7: Sensograms illustrating the consecutive and simultaneous antigen binding of TBTI-2.

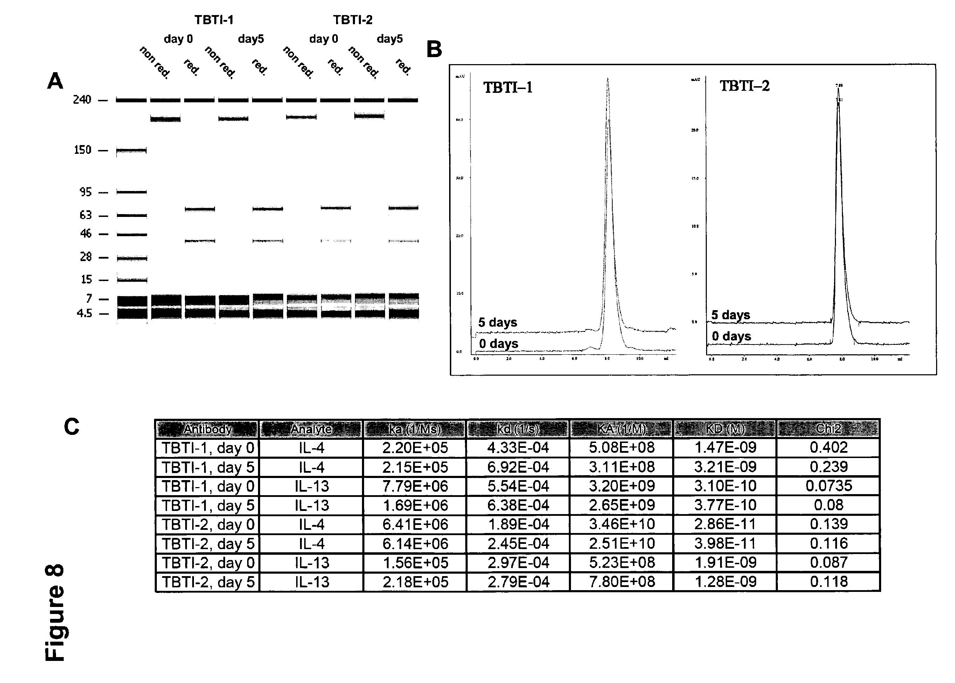

Figure 8: Illustration of the stability of the TBTI molecule after incubation in PBS at 37°C for 5 days.

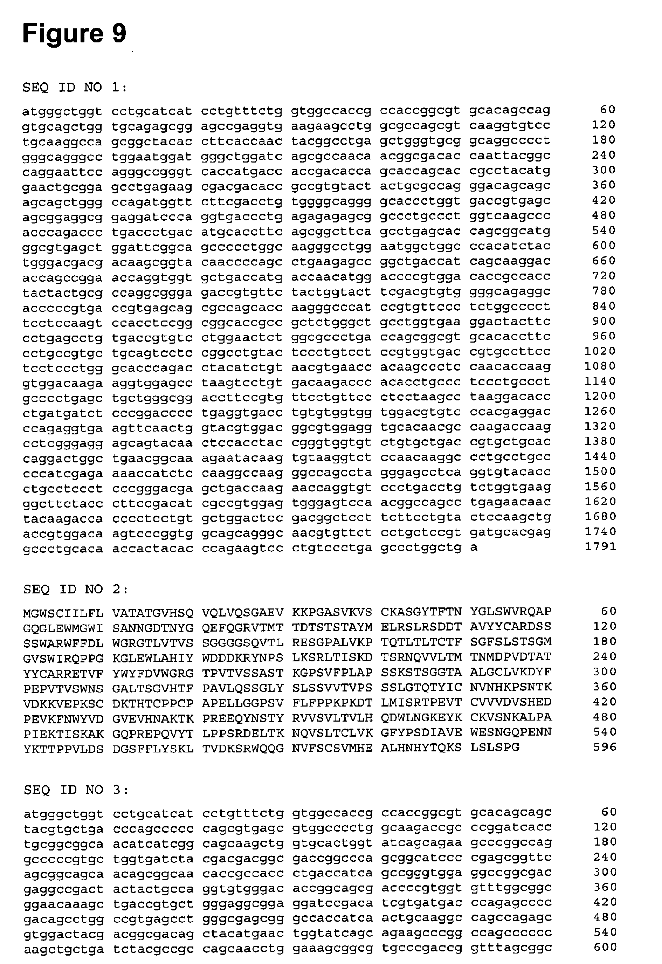

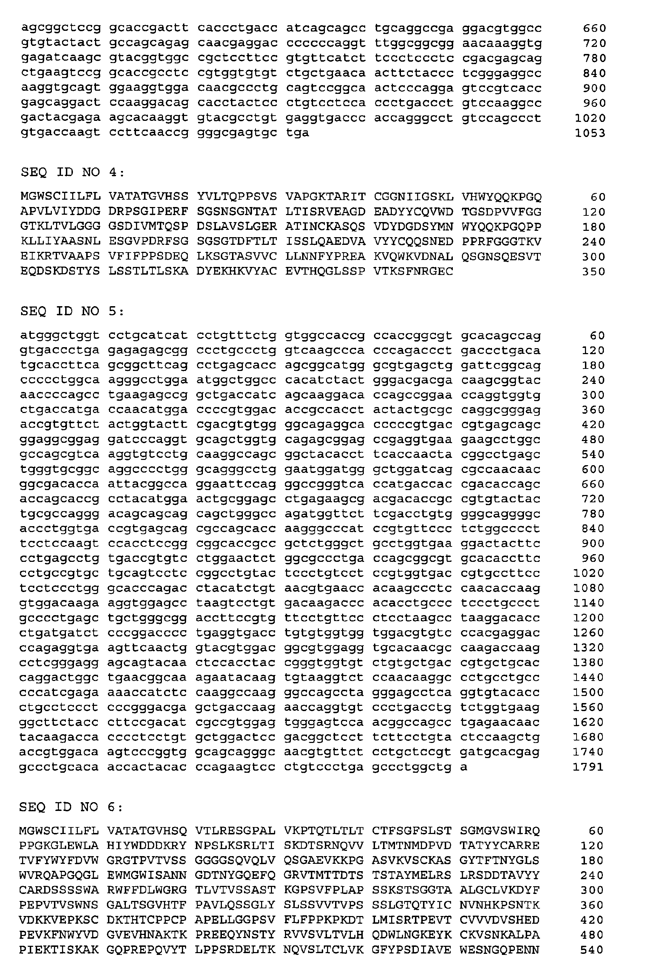

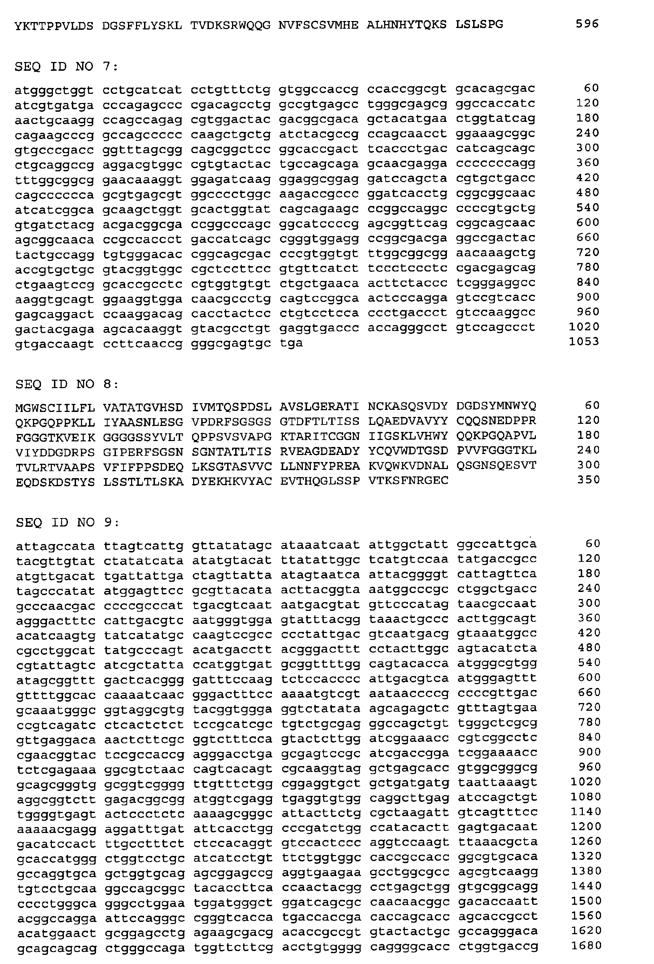

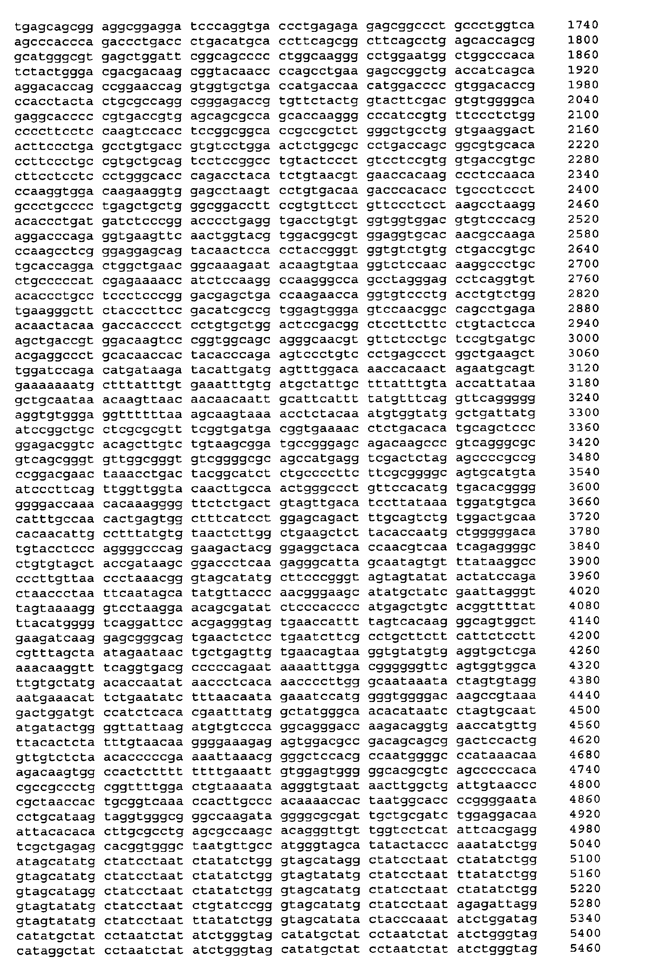

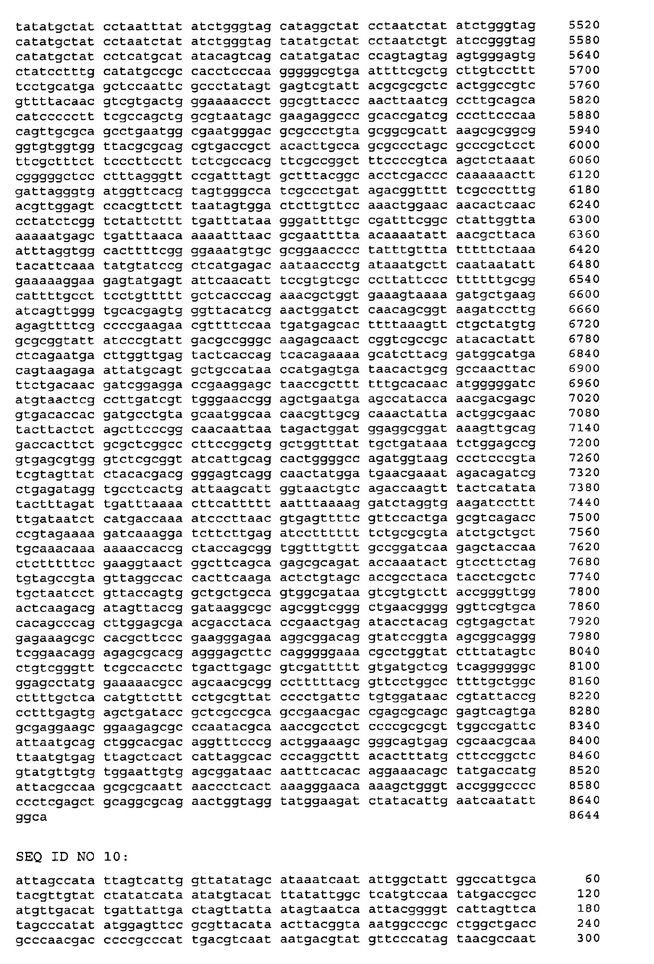

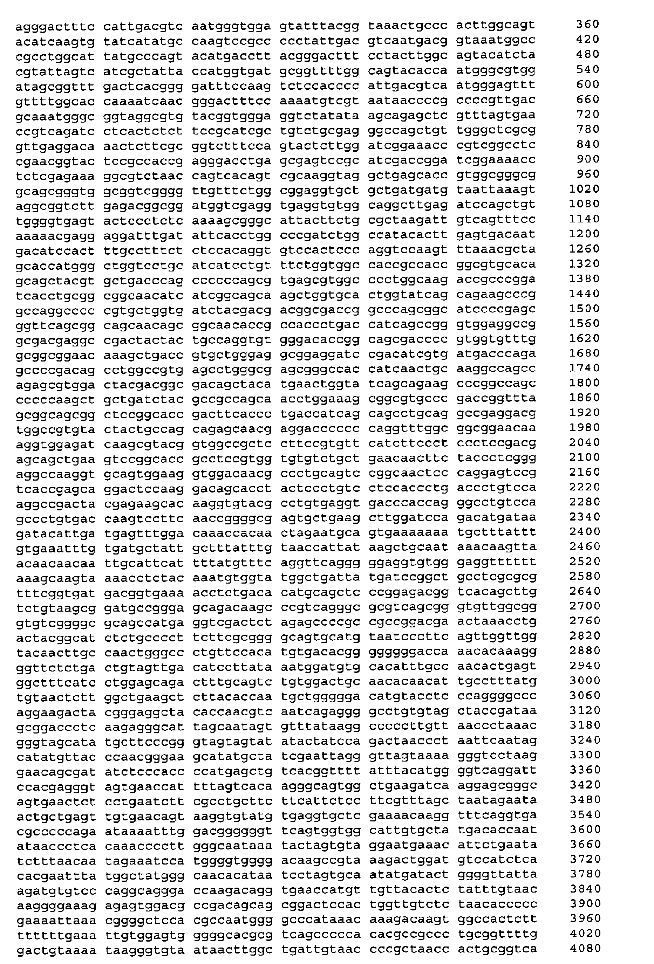

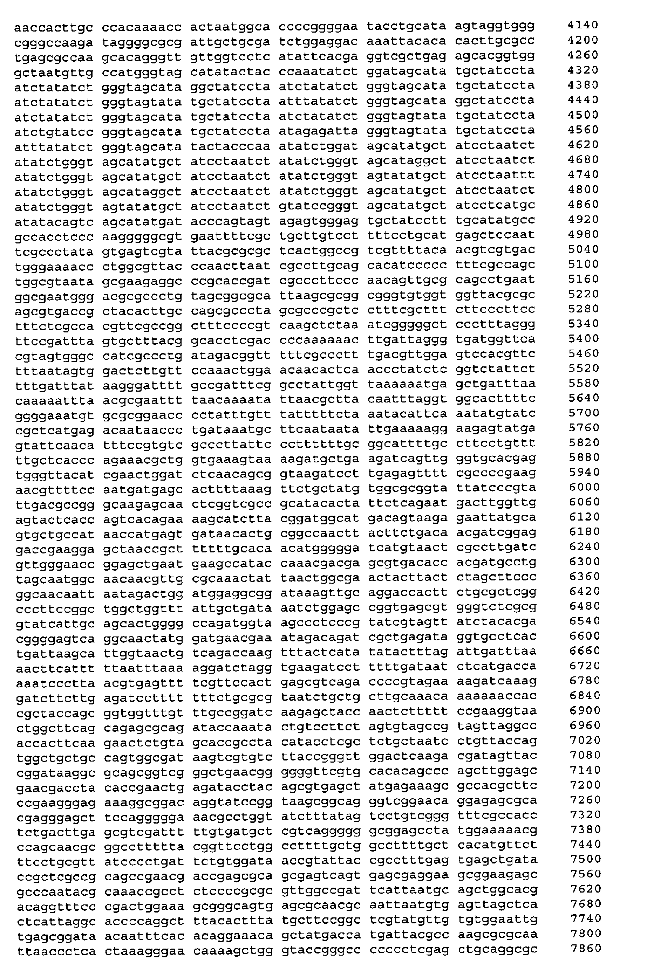

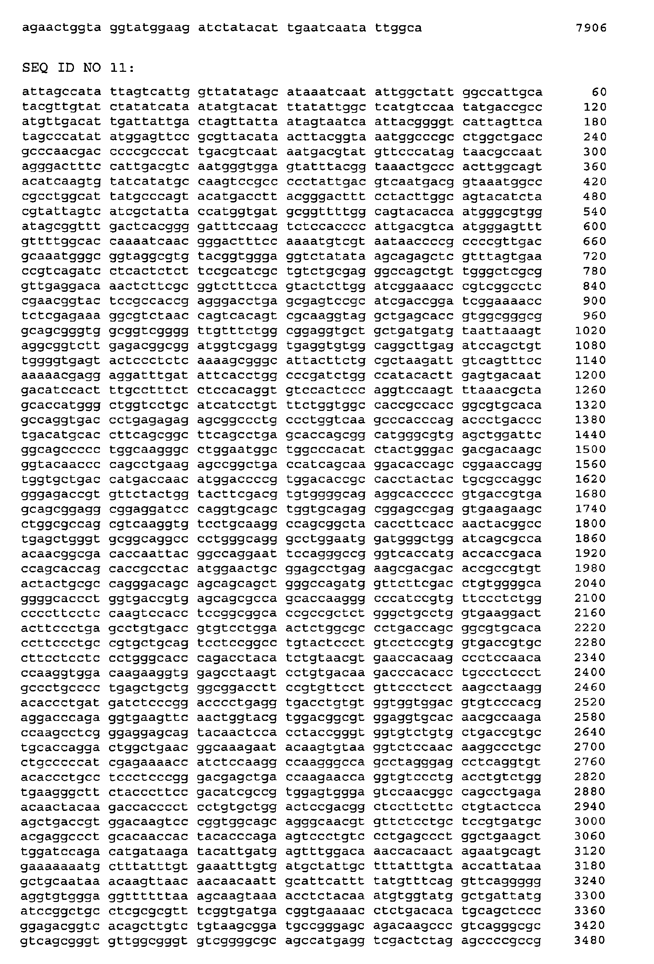

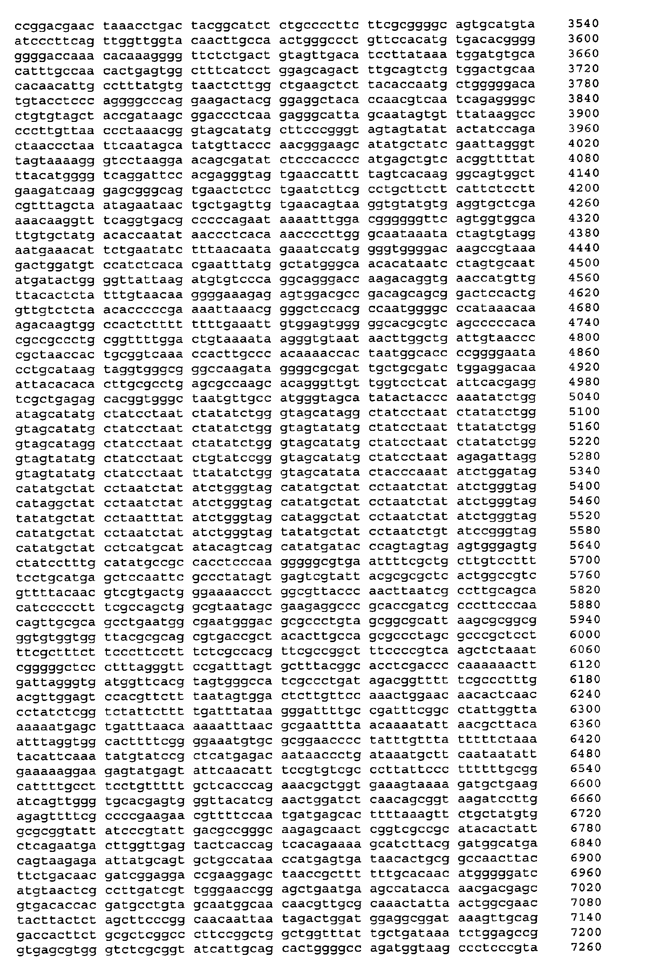

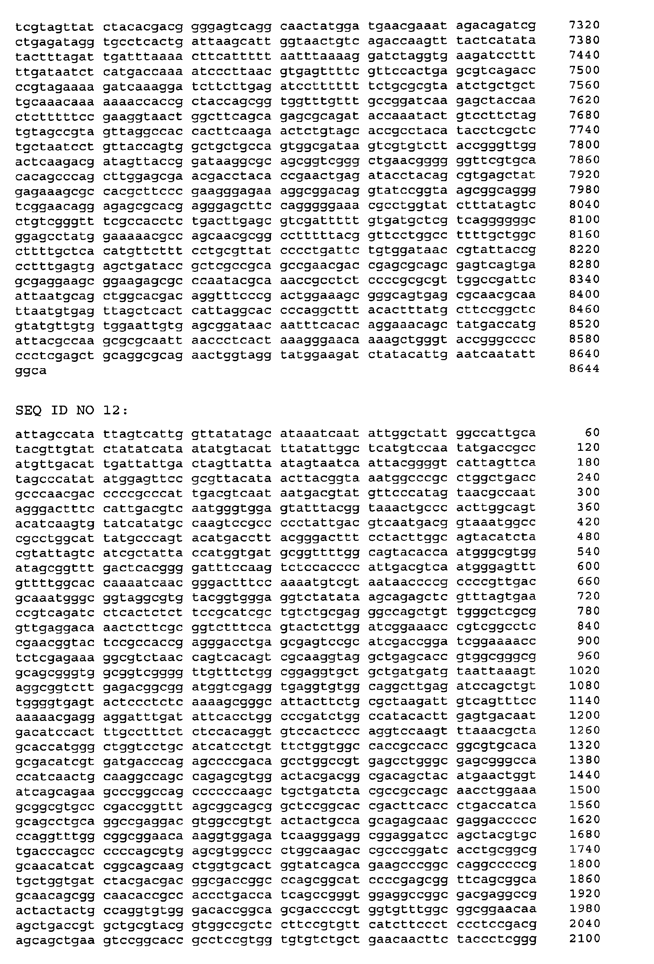

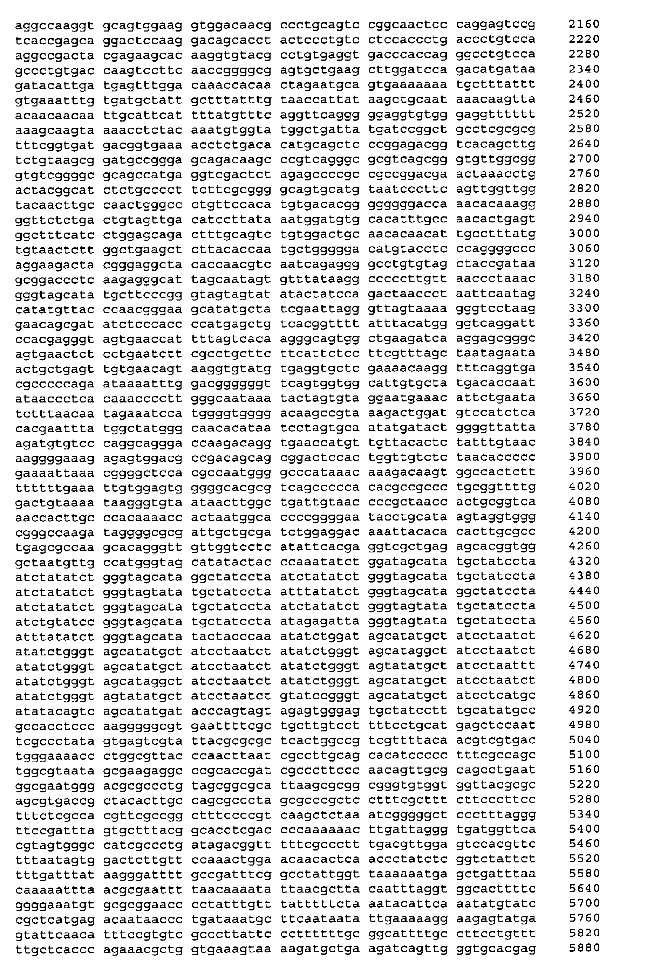

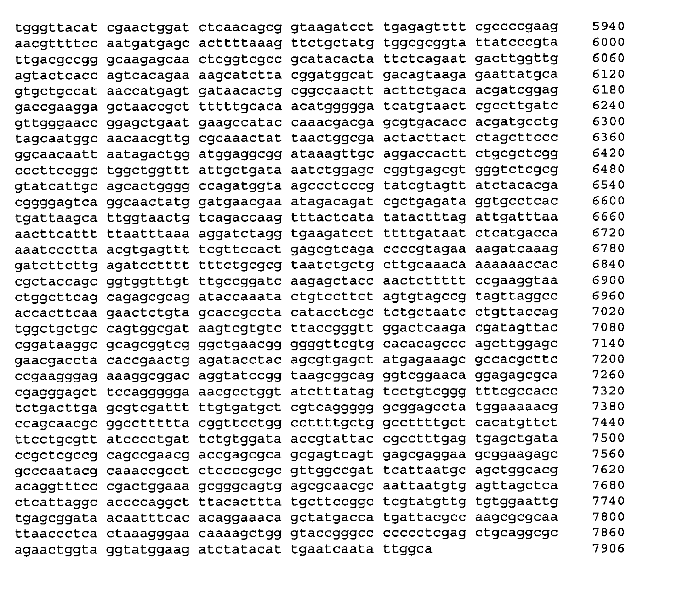

Figure 9: DNA and polypeptide sequences of TBTI1 and TBTI2

Application Project
------------------
<120> Title : Novel polyvalent bispecific antibody format and uses thereof
<130> AppFileReference : DE2007/048
<140> CurrentAppNumber :
<141> CurrentFilingDate : ____-__-__

Sequence
--------
<213> OrganismName :
<400> PreSequenceString :
atgggctggt cctgcatcat cctgtttctg gtggccaccg ccaccggcgt gcacagccag 60
gtgcagctgg tgcagagcgg agccgaggtg aagaagcctg gcgccagcgt caaggtgtcc 120
tgcaaggcca gcggctacac cttcaccaac tacggcctga gctgggtgcg gcaggcccct 180
gggcagggcc tggaatggat gggctggatc agcgccaaca acggcgacac caattacggc 240
caggaattcc agggccgggt caccatgacc accgacacca gcaccagcac cgcctacatg 300
gaactgcgga gcctgagaag cgacgacacc gccgtgtact actgcgccag ggacagcagc 360
agcagctggg ccagatggtt cttcgacctg tggggcaggg gcaccctggt gaccgtgagc 420
agcggaggcg gaggatccca ggtgaccctg agagagagcg gccctgccct ggtcaagccc 480
acccagaccc tgaccctgac atgcaccttc agcggcttca gcctgagcac cagcggcatg 540
ggcgtgagct ggattcggca gccccctggc aagggcctgg aatggctggc ccacatctac 600
tgggacgacg acaagcggta caaccccagc ctgaagagcc ggctgaccat cagcaaggac 660
accagccgga accaggtggt gctgaccatg accaacatgg accccgtgga caccgccacc 720
tactactgcg ccaggcggga gaccgtgttc tactggtact tcgacgtgtg gggcagaggc 780
acccccgtga ccgtgagcag cgccagcacc aagggcccat ccgtgttccc tctggcccct 840
tcctccaagt ccacctccgg cggcaccgcc gctctgggct gcctggtgaa ggactacttc 900
cctgagcctg tgaccgtgtc ctggaactct ggcgccctga ccagcggcgt gcacaccttc 960
cctgccgtgc tgcagtcctc cggcctgtac tccctgtcct ccgtggtgac cgtgccttcc 1020
tcctccctgg gcacccagac ctacatctgt aacgtgaacc acaagccctc caacaccaag 1080
gtggacaaga aggtggagcc taagtcctgt gacaagaccc acacctgccc tccctgccct 1140
gcccctgagc tgctgggcgg accttccgtg ttcctgttcc ctcctaagcc taaggacacc 1200
ctgatgatct cccggacccc tgaggtgacc tgtgtggtgg tggacgtgtc ccacgaggac 1260
ccagaggtga agttcaactg gtacgtggac ggcgtggagg tgcacaacgc caagaccaag 1320
cctcgggagg agcagtacaa ctccacctac cgggtggtgt ctgtgctgac cgtgctgcac 1380
caggactggc tgaacggcaa agaatacaag tgtaaggtct ccaacaaggc cctgcctgcc 1440
cccatcgaga aaaccatctc caaggccaag ggccagccta gggagcctca ggtgtacacc 1500
ctgcctccct cccgggacga gctgaccaag aaccaggtgt ccctgacctg tctggtgaag 1560
ggcttctacc cttccgacat cgccgtggag tgggagtcca acggccagcc tgagaacaac 1620
tacaagacca cccctcctgt gctggactcc gacggctcct tcttcctgta ctccaagctg 1680
accgtggaca agtcccggtg gcagcagggc aacgtgttct cctgctccgt gatgcacgag 1740
gccctgcaca accactacac ccagaagtcc ctgtccctga gccctggctg a 1791

<212> Type : DNA
<211> Length : 1791
SequenceName : SEQ ID NO 1
SequenceDescription :

Sequence
--------
<213> OrganismName :
<400> PreSequenceString :
MGWSCIILFL VATATGVHSQ VQLVQSGAEV KKPGASVKVS CKASGYTFTN YGLSWVRQAP 60
GQGLEWMGWI SANNGDTNYG QEFQGRVTMT TDTSTSTAYM ELRSLRSDDT AVYYCARDSS 120
SSWARWFFDL WGRGTLVTVS SGGGGSQVTL RESGPALVKP TQTLTLTCTF SGFSLSTSGM 180
GVSWIRQPPG KGLEWLAHIY WDDDKRYNPS LKSRLTISKD TSRNQVVLTM TNMDPVDTAT 240
YYCARRETVF YWYFDVWGRG TPVTVSSAST KGPSVFPLAP SSKSTSGGTA ALGCLVKDYF 300
PEPVTVSWNS GALTSGVHTF PAVLQSSGLY SLSSVVTVPS SSLGTQTYIC NVNHKPSNTK 360
VDKKVEPKSC DKTHTCPPCP APELLGGPSV FLFPPKPKDT LMISRTPEVT CVVVDVSHED 420
PEVKFNWYVD GVEVHNAKTK PREEQYNSTY RVVSVLTVLH QDWLNGKEYK CKVSNKALPA 480
PIEKTISKAK GQPREPQVYT LPPSRDELTK NQVSLTCLVK GFYPSDIAVE WESNGQPENN 540
YKTTPPVLDS DGSFFLYSKL TVDKSRWQQG NVFSCSVMHE ALHNHYTQKS LSLSPG 596

<212> Type : PRT
<211> Length : 596
SequenceName : SEQ ID NO 2
SequenceDescription :

Sequence

--------
<213> OrganismName :
<400> PreSequenceString :
atgggctggt cctgcatcat cctgtttctg gtggccaccg ccaccggcgt gcacagcagc 60
tacgtgctga cccagccccc cagcgtgagc gtggcccctg gcaagaccgc ccggatcacc 120
tgcggcggca acatcatcgg cagcaagctg gtgcactggt atcagcagaa gcccggccag 180
gcccccgtgc tggtgatcta cgacgacggc gaccggccca gcggcatccc cgagcggttc 240
agcggcagca acagcggcaa caccgccacc ctgaccatca gccgggtgga ggccggcgac 300
gaggccgact actactgcca ggtgtgggac accggcagcg accccgtggt gtttggcggc 360
ggaacaaagc tgaccgtgct gggaggcgga ggatccgaca tcgtgatgac ccagagcccc 420
gacagcctgg ccgtgagcct gggcgagcgg gccaccatca actgcaaggc cagccagagc 480
gtggactacg acggcgacag ctacatgaac tggtatcagc agaagcccgg ccagcccccc 540
aagctgctga tctacgccgc cagcaacctg gaaagcggcg tgcccgaccg gtttagcggc 600
agcggctccg gcaccgactt caccctgacc atcagcagcc tgcaggccga ggacgtggcc 660
gtgtactact gccagcagag caacgaggac ccccccaggt ttggcggcgg aacaaaggtg 720
gagatcaagc gtacggtggc cgctccttcc gtgttcatct tccctccctc cgacgagcag 780
ctgaagtccg gcaccgcctc cgtggtgtgt ctgctgaaca acttctaccc tcgggaggcc 840
aaggtgcagt ggaaggtgga caacgccctg cagtccggca actcccagga gtccgtcacc 900
gagcaggact ccaaggacag cacctactcc ctgtcctcca ccctgaccct gtccaaggcc 960
gactacgaga agcacaaggt gtacgcctgt gaggtgaccc accagggcct gtccagccct 1020
gtgaccaagt ccttcaaccg gggcgagtgc tga 1053
<212> Type : DNA
<211> Length : 1053
SequenceName : SEQ ID NO 3
SequenceDescription :

Sequence
--------
<213> OrganismName :
<400> PreSequenceString :
MGWSCIILFL VATATGVHSS YVLTQPPSVS VAPGKTARIT CGGNIIGSKL VHWYQQKPGQ 60
APVLVIYDDG DRPSGIPERF SGSNSGNTAT LTISRVEAGD EADYYCQVWD TGSDPVVFGG 120
GTKLTVLGGG GSDIVMTQSP DSLAVSLGER ATINCKASQS VDYDGDSYMN WYQQKPGQPP 180
KLLIYAASNL ESGVPDRFSG SGSGTDFTLT ISSLQAEDVA VYYCQQSNED PPRFGGGTKV 240
EIKRTVAAPS VFIFPPSDEQ LKSGTASVVC LLNNFYPREA KVQWKVDNAL QSGNSQESVT 300
EQDSKDSTYS LSSTLTLSKA DYEKHKVYAC EVTHQGLSSP VTKSFNRGEC 350
<212> Type : PRT
<211> Length : 350
SequenceName : SEQ ID NO 4
SequenceDescription :

Sequence
--------
<213> OrganismName :
<400> PreSequenceString :
atgggctggt cctgcatcat cctgtttctg gtggccaccg ccaccggcgt gcacagccag 60
gtgaccctga gagagagcgg ccctgccctg gtcaagccca cccagaccct gaccctgaca 120
tgcaccttca gcggcttcag cctgagcacc agcggcatgg gcgtgagctg gattcggcag 180
ccccctggca agggcctgga atggctggcc cacatctact gggacgacga caagcggtac 240
aaccccagcc tgaagagccg gctgaccatc agcaaggaca ccagccggaa ccaggtggtg 300
ctgaccatga ccaacatgga ccccgtggac accgccacct actactgcgc caggcgggag 360
accgtgttct actggtactt cgacgtgtgg ggcagaggca cccccgtgac cgtgagcagc 420
ggaggcggag gatcccaggt gcagctggtg cagagcggag ccgaggtgaa gaagcctggc 480
gccagcgtca aggtgtcctg caaggccagc ggctacacct tcaccaacta cggcctgagc 540
tgggtgcggc aggcccctgg gcagggcctg gaatggatgg gctggatcag cgccaacaac 600
ggcgacacca attacggcca ggaattccag ggccgggtca ccatgaccac cgacaccagc 660
accagcaccg cctacatgga actgcggagc ctgagaagcg acgacaccgc cgtgtactac 720
tgcgccaggg acagcagcag cagctgggcc agatggttct tcgacctgtg gggcaggggc 780
accctggtga ccgtgagcag cgccagcacc aagggcccat ccgtgttccc tctggcccct 840
tcctccaagt ccacctccgg cggcaccgcc gctctgggct gcctggtgaa ggactacttc 900
cctgagcctg tgaccgtgtc ctggaactct ggcgccctga ccagcggcgt gcacaccttc 960
cctgccgtgc tgcagtcctc cggcctgtac tccctgtcct ccgtggtgac cgtgccttcc 1020
tcctccctgg gcacccagac ctacatctgt aacgtgaacc acaagccctc caacaccaag 1080
gtggacaaga aggtggagcc taagtcctgt gacaagaccc acacctgccc tccctgccct 1140
gcccctgagc tgctgggcgg accttccgtg ttcctgttcc ctcctaagcc taaggacacc 1200
ctgatgatct cccggacccc tgaggtgacc tgtgtggtgg tggacgtgtc ccacgaggac 1260
ccagaggtga agttcaactg gtacgtggac ggcgtggagg tgcacaacgc caagaccaag 1320
cctcgggagg agcagtacaa ctccacctac cgggtggtgt ctgtgctgac cgtgctgcac 1380

caggactggc tgaacggcaa agaatacaag tgtaaggtct ccaacaaggc cctgcctgcc 1440
cccatcgaga aaaccatctc caaggccaag ggccagccta gggagcctca ggtgtacacc 1500
ctgcctccct cccgggacga gctgaccaag aaccaggtgt ccctgacctg tctggtgaag 1560
ggcttctacc cttccgacat cgccgtggag tgggagtcca acggccagcc tgagaacaac 1620
tacaagacca cccctcctgt gctggactcc gacggctcct tcttcctgta ctccaagctg 1680
accgtggaca agtcccggtg gcagcagggc aacgtgttct cctgctccgt gatgcacgag 1740
gccctgcaca accactacac ccagaagtcc ctgtccctga gccctggctg a 1791

<212> Type : DNA
<211> Length : 1791
SequenceName : SEQ ID NO 5
SequenceDescription :

Sequence
--------
<213> OrganismName :
<400> PreSequenceString :
MGWSCIILFL VATATGVHSQ VTLRESGPAL VKPTQTLTLT CTFSGFSLST SGMGVSWIRQ 60
PPGKGLEWLA HIYWDDDKRY NPSLKSRLTI SKDTSRNQVV LTMTNMDPVD TATYYCARRE 120
TVFYWYFDVW GRGTPVTVSS GGGGSQVQLV QSGAEVKKPG ASVKVSCKAS GYTFTNYGLS 180
WVRQAPGQGL EWMGWISANN GDTNYGQEFQ GRVTMTTDTS TSTAYMELRS LRSDDTAVYY 240
CARDSSSSWA RWFFDLWGRG TLVTVSSAST KGPSVFPLAP SSKSTSGGTA ALGCLVKDYF 300
PEPVTVSWNS GALTSGVHTF PAVLQSSGLY SLSSVVTVPS SSLGTQTYIC NVNHKPSNTK 360
VDKKVEPKSC DKTHTCPPCP APELLGGPSV FLFPPKPKDT LMISRTPEVT CVVVDVSHED 420
PEVKFNWYVD GVEVHNAKTK PREEQYNSTY RVVSVLTVLH QDWLNGKEYK CKVSNKALPA 480
PIEKTISKAK GQPREPQVYT LPPSRDELTK NQVSLTCLVK GFYPSDIAVE WESNGQPENN 540
YKTTPPVLDS DGSFFLYSKL TVDKSRWQQG NVFSCSVMHE ALHNHYTQKS LSLSPG 596

<212> Type : PRT
<211> Length : 596
SequenceName : SEQ ID NO 6
SequenceDescription :

Sequence
--------
<213> OrganismName :
<400> PreSequenceString :
atgggctggt cctgcatcat cctgtttctg gtggccaccg ccaccggcgt gcacagcgac 60
atcgtgatga cccagagccc cgacagcctg gccgtgagcc tgggcgagcg ggccaccatc 120
aactgcaagg ccagccagag cgtggactac gacggcgaca gctacatgaa ctggtatcag 180
cagaagcccg gccagccccc caagctgctg atctacgccg ccagcaacct ggaaagcggc 240
gtgcccgacc ggtttagcgg cagcggctcc ggcaccgact tcaccctgac catcagcagc 300
ctgcaggccg aggacgtggc cgtgtactac tgccagcaga gcaacgagga cccccccagg 360
tttggcggcg gaacaaaggt ggagatcaag ggaggcggag gatccagcta cgtgctgacc 420
cagcccccca gcgtgagcgt ggcccctggc aagaccgccc ggatcacctg cggcggcaac 480
atcatcggca gcaagctggt gcactggtat cagcagaagc ccggccaggc ccccgtgctg 540
gtgatctacg acgacggcga ccggcccagc ggcatccccg agcggttcag cggcagcaac 600
agcggcaaca ccgccaccct gaccatcagc cgggtggagg ccggcgacga ggccgactac 660
tactgccagg tgtgggacac cggcagcgac cccgtggtgt ttggcggcgg aacaaagctg 720
accgtgctgc gtacggtggc cgctccttcc gtgttcatct tccctccctc cgacgagcag 780
ctgaagtccg gcaccgcctc cgtggtgtgt ctgctgaaca acttctaccc tcgggaggcc 840
aaggtgcagt ggaaggtgga caacgccctg cagtccggca actcccagga gtccgtcacc 900
gagcaggact ccaaggacag cacctactcc ctgtcctcca ccctgaccct gtccaaggcc 960
gactacgaga agcacaaggt gtacgcctgt gaggtgaccc accagggcct gtccagccct 1020
gtgaccaagt ccttcaaccg gggcgagtgc tga 1053
<212> Type : DNA
<211> Length : 1053
SequenceName : SEQ ID NO 7
SequenceDescription :

Sequence
--------
<213> OrganismName :
<400> PreSequenceString :
MGWSCIILFL VATATGVHSD IVMTQSPDSL AVSLGERATI NCKASQSVDY DGDSYMNWYQ 60
QKPGQPPKLL IYAASNLESG VPDRFSGSGS GTDFTLTISS LQAEDVAVYY CQQSNEDPPR 120
FGGGTKVEIK GGGGSSYVLT QPPSVSVAPG KTARITCGGN IIGSKLVHWY QQKPGQAPVL 180
VIYDDGDRPS GIPERFSGSN SGNTATLTIS RVEAGDEADY YCQVWDTGSD PVVFGGGTKL 240

TVLRTVAAPS VFIFPPSDEQ LKSGTASVVC LLNNFYPREA KVQWKVDNAL QSGNSQESVT 300
EQDSKDSTYS LSSTLTLSKA DYEKHKVYAC EVTHQGLSSP VTKSFNRGEC 350
<212> Type : PRT
<211> Length : 350
SequenceName : SEQ ID NO 8
SequenceDescription :

Sequence
--------
<213> OrganismName :
<400> PreSequenceString :
attagccata ttagtcattg gttatatagc ataaatcaat attggctatt ggccattgca 60
tacgttgtat ctatatcata atatgtacat ttatattggc tcatgtccaa tatgaccgcc 120
atgttgacat tgattattga ctagttatta atagtaatca attacggggt cattagttca 180
tagcccatat atggagttcc gcgttacata acttacggta aatggcccgc ctggctgacc 240
gcccaacgac ccccgcccat tgacgtcaat aatgacgtat gttcccatag taacgccaat 300
agggactttc cattgacgtc aatgggtgga gtatttacgg taaactgccc acttggcagt 360
acatcaagtg tatcatatgc caagtccgcc ccctattgac gtcaatgacg gtaaatggcc 420
cgcctggcat tatgcccagt acatgacctt acgggacttt cctacttggc agtacatcta 480
cgtattagtc atcgctatta ccatggtgat gcggttttgg cagtacacca atgggcgtgg 540
atagcggttt gactcacggg gatttccaag tctccacccc attgacgtca atgggagttt 600
gttttggcac caaaatcaac gggactttcc aaaatgtcgt aataaccccg ccccgttgac 660
gcaaatgggc ggtaggcgtg tacggtggga ggtctatata agcagagctc gtttagtgaa 720
ccgtcagatc ctcactctct tccgcatcgc tgtctgcgag ggccagctgt tgggctcgcg 780
gttgaggaca aactcttcgc ggtctttcca gtactcttgg atcggaaacc cgtcggcctc 840
cgaacggtac tccgccaccg agggacctga gcgagtccgc atcgaccgga tcggaaaacc 900
tctcgagaaa ggcgtctaac cagtcacagt cgcaaggtag gctgagcacc gtggcgggcg 960
gcagcgggtg gcggtcgggg ttgtttctgg cggaggtgct gctgatgatg taattaaagt 1020
aggcggtctt gagacggcgg atggtcgagg tgaggtgtgg caggcttgag atccagctgt 1080
tggggtgagt actccctctc aaaagcgggc attacttctg cgctaagatt gtcagtttcc 1140
aaaaacgagg aggatttgat attcacctgg cccgatctgg ccatacactt gagtgacaat 1200
gacatccact ttgcctttct ctccacaggt gtccactccc aggtccaagt ttaaacgcta 1260
gcaccatggg ctggtcctgc atcatcctgt ttctggtggc caccgccacc ggcgtgcaca 1320
gccaggtgca gctggtgcag agcggagccg aggtgaagaa gcctggcgcc agcgtcaagg 1380
tgtcctgcaa ggccagcggc tacaccttca ccaactacgg cctgagctgg gtgcggcagg 1440
cccctgggca gggcctggaa tggatgggct ggatcagcgc caacaacggc gacaccaatt 1500
acggccagga attccagggc cgggtcacca tgaccaccga caccagcacc agcaccgcct 1560
acatggaact gcggagcctg agaagcgacg acaccgccgt gtactactgc gccagggaca 1620
gcagcagcag ctgggccaga tggttcttcg acctgtgggg caggggcacc ctggtgaccg 1680
tgagcagcgg aggcggagga tcccaggtga ccctgagaga gagcggccct gccctggtca 1740
agcccaccca gaccctgacc ctgacatgca ccttcagcgg cttcagcctg agcaccagcg 1800
gcatgggcgt gagctggatt cggcagcccc ctggcaaggg cctggaatgg ctggcccaca 1860
tctactggga cgacgacaag cggtacaacc ccagcctgaa gagccggctg accatcagca 1920
aggacaccag ccggaaccag gtggtgctga ccatgaccaa catggacccc gtggacaccg 1980
ccacctacta ctgcgccagg cgggagaccg tgttctactg gtacttcgac gtgtggggca 2040
gaggcacccc cgtgaccgtg agcagcgcca gcaccaaggg cccatccgtg ttccctctgg 2100
ccccttcctc caagtccacc tccggcggca ccgccgctct gggctgcctg gtgaaggact 2160
acttccctga gcctgtgacc gtgtcctgga actctggcgc cctgaccagc ggcgtgcaca 2220
ccttccctgc cgtgctgcag tcctccggcc tgtactccct gtcctccgtg gtgaccgtgc 2280
cttcctcctc cctgggcacc cagacctaca tctgtaacgt gaaccacaag ccctccaaca 2340
ccaaggtgga caagaaggtg gagcctaagt cctgtgacaa gacccacacc tgccctccct 2400
gccctgcccc tgagctgctg ggcggacctt ccgtgttcct gttccctcct aagcctaagg 2460
acaccctgat gatctcccgg acccctgagg tgacctgtgt ggtggtggac gtgtcccacg 2520
aggacccaga ggtgaagttc aactggtacg tggacggcgt ggaggtgcac aacgccaaga 2580
ccaagcctcg ggaggagcag tacaactcca cctaccgggt ggtgtctgtg ctgaccgtgc 2640
tgcaccagga ctggctgaac ggcaaagaat acaagtgtaa ggtctccaac aaggccctgc 2700
ctgcccccat cgagaaaacc atctccaagg ccaagggcca gcctagggag cctcaggtgt 2760
acaccctgcc tccctcccgg gacgagctga ccaagaacca ggtgtccctg acctgtctgg 2820
tgaagggctt ctacccttcc gacatcgccg tggagtggga gtccaacggc cagcctgaga 2880
acaactacaa gaccacccct cctgtgctgg actccgacgg ctccttcttc ctgtactcca 2940
agctgaccgt ggacaagtcc cggtggcagc agggcaacgt gttctcctgc tccgtgatgc 3000
acgaggccct gcacaaccac tacacccaga agtccctgtc cctgagccct ggctgaagct 3060
tggatccaga catgataaga tacattgatg agtttggaca aaccacaact agaatgcagt 3120
gaaaaaaatg ctttatttgt gaaatttgtg atgctattgc tttatttgta accattataa 3180
gctgcaataa acaagttaac aacaacaatt gcattcattt tatgtttcag gttcaggggg 3240
aggtgtggga ggttttttaa agcaagtaaa acctctacaa atgtggtatg gctgattatg 3300
atccggctgc ctcgcgcgtt tcggtgatga cggtgaaaac ctctgacaca tgcagctccc 3360
ggagacggtc acagcttgtc tgtaagcgga tgccgggagc agacaagccc gtcagggcgc 3420

gtcagcgggt gttggcgggt gtcggggcgc agccatgagg tcgactctag agccccgccg 3480
ccggacgaac taaacctgac tacggcatct ctgccccttc ttcgcggggc agtgcatgta 3540
atcccttcag ttggttggta caacttgcca actgggccct gttccacatg tgacacgggg 3600
ggggaccaaa cacaaagggg ttctctgact gtagttgaca tccttataaa tggatgtgca 3660
catttgccaa cactgagtgg ctttcatcct ggagcagact ttgcagtctg tggactgcaa 3720
cacaacattg cctttatgtg taactcttgg ctgaagctct tacaccaatg ctgggggaca 3780
tgtacctccc aggggcccag gaagactacg ggaggctaca ccaacgtcaa tcagaggggc 3840
ctgtgtagct accgataagc ggaccctcaa gagggcatta gcaatagtgt ttataaggcc 3900
cccttgttaa ccctaaacgg gtagcatatg cttcccgggt agtagtatat actatccaga 3960
ctaaccctaa ttcaatagca tatgttaccc aacgggaagc atatgctatc gaattagggt 4020
tagtaaaagg gtcctaagga acagcgatat ctcccacccc atgagctgtc acggttttat 4080
ttacatgggg tcaggattcc acgagggtag tgaaccattt tagtcacaag ggcagtggct 4140
gaagatcaag gagcgggcag tgaactctcc tgaatcttcg cctgcttctt cattctcctt 4200
cgtttagcta atagaataac tgctgagttg tgaacagtaa ggtgtatgtg aggtgctcga 4260
aaacaaggtt tcaggtgacg cccccagaat aaaatttgga cggggggttc agtggtggca 4320
ttgtgctatg acaccaatat aaccctcaca aaccccttgg gcaataaata ctagtgtagg 4380
aatgaaacat tctgaatatc tttaacaata gaaatccatg gggtggggac aagccgtaaa 4440
gactggatgt ccatctcaca cgaatttatg gctatgggca acacataatc ctagtgcaat 4500
atgatactgg ggttattaag atgtgtccca ggcagggacc aagacaggtg aaccatgttg 4560
ttacactcta tttgtaacaa ggggaaagag agtggacgcc gacagcagcg gactccactg 4620
gttgtctcta acacccccga aaattaaacg gggctccacg ccaatggggc ccataaacaa 4680
agacaagtgg ccactctttt ttttgaaatt gtggagtggg ggcacgcgtc agcccccaca 4740
cgccgccctg cggttttgga ctgtaaaata agggtgtaat aacttggctg attgtaaccc 4800
cgctaaccac tgcggtcaaa ccacttgccc acaaaaccac taatggcacc ccggggaata 4860
cctgcataag taggtgggcg ggccaagata ggggcgcgat tgctgcgatc tggaggacaa 4920
attacacaca cttgcgcctg agcgccaagc acagggttgt tggtcctcat attcacgagg 4980
tcgctgagag cacggtgggc taatgttgcc atgggtagca tatactaccc aaatatctgg 5040
atagcatatg ctatcctaat ctatatctgg gtagcatagg ctatcctaat ctatatctgg 5100
gtagcatatg ctatcctaat ctatatctgg gtagtatatg ctatcctaat ttatatctgg 5160
gtagcatagg ctatcctaat ctatatctgg gtagcatatg ctatcctaat ctatatctgg 5220
gtagtatatg ctatcctaat ctgtatccgg gtagcatatg ctatcctaat agagattagg 5280
gtagtatatg ctatcctaat ttatatctgg gtagcatata ctacccaaat atctggatag 5340
catatgctat cctaatctat atctgggtag catatgctat cctaatctat atctgggtag 5400
cataggctat cctaatctat atctgggtag catatgctat cctaatctat atctgggtag 5460
tatatgctat cctaatttat atctgggtag cataggctat cctaatctat atctgggtag 5520
catatgctat cctaatctat atctgggtag tatatgctat cctaatctgt atccgggtag 5580
catatgctat cctcatgcat atacagtcag catatgatac ccagtagtag agtgggagtg 5640
ctatcctttg catatgccgc cacctcccaa gggggcgtga attttcgctg cttgtccttt 5700
tcctgcatga gctccaattc gccctatagt gagtcgtatt acgcgcgctc actggccgtc 5760
gttttacaac gtcgtgactg ggaaaaccct ggcgttaccc aacttaatcg ccttgcagca 5820
catccccctt tcgccagctg gcgtaatagc gaagaggccc gcaccgatcg cccttcccaa 5880
cagttgcgca gcctgaatgg cgaatgggac gcgccctgta gcggcgcatt aagcgcggcg 5940
ggtgtggtgg ttacgcgcag cgtgaccgct acacttgcca gcgccctagc gcccgctcct 6000
ttcgctttct tcccttcctt tctcgccacg ttcgccggct ttccccgtca agctctaaat 6060
cgggggctcc ctttagggtt ccgatttagt gctttacggc acctcgaccc caaaaaactt 6120
gattagggtg atggttcacg tagtgggcca tcgccctgat agacggtttt tcgccctttg 6180
acgttggagt ccacgttctt taatagtgga ctcttgttcc aaactggaac aacactcaac 6240
cctatctcgg tctattcttt tgatttataa gggattttgc cgatttcggc ctattggtta 6300
aaaaatgagc tgatttaaca aaaatttaac gcgaatttta acaaaatatt aacgcttaca 6360
atttaggtgg cacttttcgg ggaaatgtgc gcggaacccc tatttgttta tttttctaaa 6420
tacattcaaa tatgtatccg ctcatgagac aataaccctg ataaatgctt caataatatt 6480
gaaaaaggaa gagtatgagt attcaacatt tccgtgtcgc ccttattccc ttttttgcgg 6540
cattttgcct tcctgttttt gctcacccag aaacgctggt gaaagtaaaa gatgctgaag 6600
atcagttggg tgcacgagtg ggttacatcg aactggatct caacagcggt aagatccttg 6660
agagttttcg ccccgaagaa cgttttccaa tgatgagcac ttttaaagtt ctgctatgtg 6720
gcgcggtatt atcccgtatt gacgccgggc aagagcaact cggtcgccgc atacactatt 6780
ctcagaatga cttggttgag tactcaccag tcacagaaaa gcatcttacg gatggcatga 6840
cagtaagaga attatgcagt gctgccataa ccatgagtga taacactgcg gccaacttac 6900
ttctgacaac gatcggagga ccgaaggagc taaccgcttt tttgcacaac atgggggatc 6960
atgtaactcg ccttgatcgt tgggaaccgg agctgaatga agccatacca aacgacgagc 7020
gtgacaccac gatgcctgta gcaatggcaa caacgttgcg caaactatta actggcgaac 7080
tacttactct agcttcccgg caacaattaa tagactggat ggaggcggat aaagttgcag 7140
gaccacttct gcgctcggcc cttccggctg gctggtttat tgctgataaa tctggagccg 7200
gtgagcgtgg gtctcgcggt atcattgcag cactggggcc agatggtaag ccctcccgta 7260
tcgtagttat ctacacgacg gggagtcagg caactatgga tgaacgaaat agacagatcg 7320
ctgagatagg tgcctcactg attaagcatt ggtaactgtc agaccaagtt tactcatata 7380
tactttagat tgatttaaaa cttcattttt aatttaaaag gatctaggtg aagatccttt 7440
ttgataatct catgaccaaa atcccttaac gtgagttttc gttccactga gcgtcagacc 7500

ccgtagaaaa gatcaaagga tcttcttgag atcctttttt tctgcgcgta atctgctgct 7560
tgcaaacaaa aaaaccaccg ctaccagcgg tggtttgttt gccggatcaa gagctaccaa 7620
ctctttttcc gaaggtaact ggcttcagca gagcgcagat accaaatact gtccttctag 7680
tgtagccgta gttaggccac cacttcaaga actctgtagc accgcctaca tacctcgctc 7740
tgctaatcct gttaccagtg gctgctgcca gtggcgataa gtcgtgtctt accgggttgg 7800
actcaagacg atagttaccg gataaggcgc agcggtcggg ctgaacgggg ggttcgtgca 7860
cacagcccag cttggagcga acgacctaca ccgaactgag atacctacag cgtgagctat 7920
gagaaagcgc cacgcttccc gaagggagaa aggcggacag gtatccggta agcggcaggg 7980
tcggaacagg agagcgcacg agggagcttc cagggggaaa cgcctggtat ctttatagtc 8040
ctgtcgggtt tcgccacctc tgacttgagc gtcgattttt gtgatgctcg tcaggggggc 8100
ggagcctatg gaaaaacgcc agcaacgcgg cctttttacg gttcctggcc ttttgctggc 8160
cttttgctca catgttcttt cctgcgttat cccctgattc tgtggataac cgtattaccg 8220
cctttgagtg agctgatacc gctcgccgca gccgaacgac cgagcgcagc gagtcagtga 8280
gcgaggaagc ggaagagcgc ccaatacgca aaccgcctct ccccgcgcgt tggccgattc 8340
attaatgcag ctggcacgac aggtttcccg actggaaagc gggcagtgag cgcaacgcaa 8400
ttaatgtgag ttagctcact cattaggcac cccaggcttt acactttatg cttccggctc 8460
gtatgttgtg tggaattgtg agcggataac aatttcacac aggaaacagc tatgaccatg 8520
attacgccaa gcgcgcaatt aaccctcact aaagggaaca aaagctgggt accgggcccc 8580
ccctcgagct gcaggcgcag aactggtagg tatggaagat ctatacattg aatcaatatt 8640
ggca 8644
<212> Type : DNA
<211> Length : 8644
SequenceName : SEQ ID NO 9
SequenceDescription :

Sequence
--------
<213> OrganismName :
<400> PreSequenceString :
attagccata ttagtcattg gttatatagc ataaatcaat attggctatt ggccattgca 60
tacgttgtat ctatatcata atatgtacat ttatattggc tcatgtccaa tatgaccgcc 120
atgttgacat tgattattga ctagttatta atagtaatca attacggggt cattagttca 180
tagcccatat atggagttcc gcgttacata acttacggta aatggcccgc ctggctgacc 240
gcccaacgac ccccgcccat tgacgtcaat aatgacgtat gttcccatag taacgccaat 300
agggactttc cattgacgtc aatgggtgga gtatttacgg taaactgccc acttggcagt 360
acatcaagtg tatcatatgc caagtccgcc ccctattgac gtcaatgacg gtaaatggcc 420
cgcctggcat tatgcccagt acatgacctt acgggacttt cctacttggc agtacatcta 480
cgtattagtc atcgctatta ccatggtgat gcggttttgg cagtacacca atgggcgtgg 540
atagcggttt gactcacggg gatttccaag tctccacccc attgacgtca atgggagttt 600
gttttggcac caaaatcaac gggactttcc aaaatgtcgt aataaccccg ccccgttgac 660
gcaaatgggc ggtaggcgtg tacggtggga ggtctatata agcagagctc gtttagtgaa 720
ccgtcagatc ctcactctct tccgcatcgc tgtctgcgag ggccagctgt tgggctcgcg 780
gttgaggaca aactcttcgc ggtctttcca gtactcttgg atcggaaacc cgtcggcctc 840
cgaacggtac tccgccaccg agggacctga gcgagtccgc atcgaccgga tcggaaaacc 900
tctcgagaaa ggcgtctaac cagtcacagt cgcaaggtag gctgagcacc gtggcgggcg 960
gcagcgggtg gcggtcgggg ttgtttctgg cggaggtgct gctgatgatg taattaaagt 1020
aggcggtctt gagacggcgg atggtcgagg tgaggtgtgg caggcttgag atccagctgt 1080
tggggtgagt actccctctc aaaagcgggc attacttctg cgctaagatt gtcagtttcc 1140
aaaaacgagg aggatttgat attcacctgg cccgatctgg ccatacactt gagtgacaat 1200
gacatccact ttgcctttct ctccacaggt gtccactccc aggtccaagt ttaaacgcta 1260
gcaccatggg ctggtcctgc atcatcctgt ttctggtggc caccgccacc ggcgtgcaca 1320
gcagctacgt gctgacccag ccccccagcg tgagcgtggc ccctggcaag accgcccgga 1380
tcacctgcgg cggcaacatc atcggcagca agctggtgca ctggtatcag cagaagcccg 1440
gccaggcccc cgtgctggtg atctacgacg acggcgaccg gcccagcggc atccccgagc 1500
ggttcagcgg cagcaacagc ggcaacaccg ccaccctgac catcagccgg gtggaggccg 1560
gcgacgaggc cgactactac tgccaggtgt gggacaccgg cagcgacccc gtggtgtttg 1620
gcggcggaac aaagctgacc gtgctgggag gcggaggatc cgacatcgtg atgacccaga 1680
gccccgacag cctggccgtg agcctgggcg agcgggccac catcaactgc aaggccagcc 1740
agagcgtgga ctacgacggc gacagctaca tgaactggta tcagcagaag cccggccagc 1800
cccccaagct gctgatctac gccgccagca acctggaaag cggcgtgccc gaccggttta 1860
gcggcagcgg ctccggcacc gacttcaccc tgaccatcag cagcctgcag gccgaggacg 1920
tggccgtgta ctactgccag cagagcaacg aggacccccc caggtttggc ggcggaacaa 1980
aggtggagat caagcgtacg gtggccgctc cttccgtgtt catcttccct ccctccgacg 2040
agcagctgaa gtccggcacc gcctccgtgg tgtgtctgct gaacaacttc taccctcggg 2100
aggccaaggt gcagtggaag gtggacaacg ccctgcagtc cggcaactcc caggagtccg 2160
tcaccgagca ggactccaag gacagcacct actccctgtc ctccaccctg accctgtcca 2220
aggccgacta cgagaagcac aaggtgtacg cctgtgaggt gacccaccag ggcctgtcca 2280
gccctgtgac caagtccttc aaccggggcg agtgctgaag cttggatcca gacatgataa 2340

gatacattga tgagtttgga caaaccacaa ctagaatgca gtgaaaaaaa tgctttattt 2400
gtgaaatttg tgatgctatt gctttatttg taaccattat aagctgcaat aaacaagtta 2460
acaacaacaa ttgcattcat tttatgtttc aggttcaggg ggaggtgtgg gaggtttttt 2520
aaagcaagta aaacctctac aaatgtggta tggctgatta tgatccggct gcctcgcgcg 2580
tttcggtgat gacggtgaaa acctctgaca catgcagctc ccggagacgg tcacagcttg 2640
tctgtaagcg gatgccggga gcagacaagc ccgtcagggc gcgtcagcgg gtgttggcgg 2700
gtgtcggggc gcagccatga ggtcgactct agagccccgc cgccggacga actaaacctg 2760
actacggcat ctctgcccct tcttcgcggg gcagtgcatg taatcccttc agttggttgg 2820
tacaacttgc caactgggcc ctgttccaca tgtgacacgg ggggggacca aacacaaagg 2880
ggttctctga ctgtagttga catccttata aatggatgtg cacatttgcc aacactgagt 2940
ggctttcatc ctggagcaga ctttgcagtc tgtggactgc aacacaacat tgcctttatg 3000
tgtaactctt ggctgaagct cttacaccaa tgctggggga catgtacctc ccaggggccc 3060
aggaagacta cgggaggcta caccaacgtc aatcagaggg gcctgtgtag ctaccgataa 3120
gcggaccctc aagagggcat tagcaatagt gtttataagg ccccccttgtt aaccctaaac 3180
gggtagcata tgcttcccgg gtagtagtat atactatcca gactaaccct aattcaatag 3240
catatgttac ccaacgggaa gcatatgcta tcgaattagg gttagtaaaa gggtcctaag 3300
gaacagcgat atctcccacc ccatgagctg tcacggtttt atttacatgg ggtcaggatt 3360
ccacgagggt agtgaaccat tttagtcaca agggcagtgg ctgaagatca aggagcgggc 3420
agtgaactct cctgaatctt cgcctgcttc ttcattctcc ttcgtttagc taatagaata 3480
actgctgagt tgtgaacagt aaggtgtatg tgaggtgctc gaaaacaagg tttcaggtga 3540
cgcccccaga ataaaatttg gacggggggt tcagtggtgg cattgtgcta tgacaccaat 3600
ataaccctca caaacccctt gggcaataaa tactagtgta ggaatgaaac attctgaata 3660
tctttaacaa tagaaatcca tggggtgggg acaagccgta aagactggat gtccatctca 3720
cacgaattta tggctatggg caacacataa tcctagtgca atatgatact ggggttatta 3780
agatgtgtcc caggcaggga ccaagacagg tgaaccatgt tgttacactc tatttgtaac 3840
aaggggaaag agagtggacg ccgacagcag cggactccac tggttgtctc taacaccccc 3900
gaaaattaaa cggggctcca cgccaatggg gcccataaac aaagacaagt ggccactctt 3960
ttttttgaaa ttgtggagtg ggggcacgcg tcagccccca cacgccgccc tgcggttttg 4020
gactgtaaaa taagggtgta ataacttggc tgattgtaac cccgctaacc actgcggtca 4080
aaccacttgc ccacaaaacc actaatggca ccccggggaa tacctgcata agtaggtggg 4140
cgggccaaga taggggcgcg attgctgcga tctggaggac aaattacaca cacttgcgcc 4200
tgagcgccaa gcacagggtt gttggtcctc atattcacga ggtcgctgag agcacggtgg 4260
gctaatgttg ccatgggtag catatactac ccaaatatct ggatagcata tgctatccta 4320
atctatatct gggtagcata ggctatccta atctatatct gggtagcata tgctatccta 4380
atctatatct gggtagtata tgctatccta atttatatct gggtagcata ggctatccta 4440
atctatatct gggtagcata tgctatccta atctatatct gggtagtata tgctatccta 4500
atctgtatcc gggtagcata tgctatccta atagagatta gggtagtata tgctatccta 4560
atttatatct gggtagcata tactacccaa atatctggat agcatatgct atcctaatct 4620
atatctgggt agcatatgct atcctaatct atatctgggt agcataggct atcctaatct 4680
atatctgggt agcatatgct atcctaatct atatctgggt agtatatgct atcctaattt 4740
atatctgggt agcataggct atcctaatct atatctgggt agcatatgct atcctaatct 4800
atatctgggt agtatatgct atcctaatct gtatccgggt agcatatgct atcctcatgc 4860
atatacagtc agcatatgat acccagtagt agagtgggag tgctatcctt tgcatatgcc 4920
gccacctccc aagggggcgt gaattttcgc tgcttgtcct tttcctgcat gagctccaat 4980
tcgccctata gtgagtcgta ttacgcgcgc tcactggccg tcgttttaca acgtcgtgac 5040
tgggaaaacc ctggcgttac ccaacttaat cgccttgcag cacatccccc tttcgccagc 5100
tggcgtaata gcgaagaggc ccgcaccgat cgcccttccc aacagttgcg cagcctgaat 5160
ggcgaatggg acgcgccctg tagcggcgca ttaagcgcgg cgggtgtggt ggttacgcgc 5220
agcgtgaccg ctacacttgc cagcgcccta gcgcccgctc ctttcgcttt cttcccttcc 5280
tttctcgcca cgttcgccgg ctttccccgt caagctctaa atcgggggct ccctttaggg 5340
ttccgattta gtgctttacg gcacctcgac cccaaaaaac ttgattaggg tgatggttca 5400
cgtagtgggc catcgccctg atagacggtt tttcgccctt tgacgttgga gtccacgttc 5460
tttaatagtg gactcttgtt ccaaactgga acaacactca accctatctc ggtctattct 5520
tttgatttat aagggatttt gccgatttcg gcctattggt taaaaaatga gctgatttaa 5580
caaaaattta acgcgaattt taacaaaata ttaacgctta caatttaggt ggcacttttc 5640
ggggaaatgt gcgcggaacc cctatttgtt tatttttcta aatacattca aatatgtatc 5700
cgctcatgag acaataaccc tgataaatgc ttcaataata ttgaaaaagg aagagtatga 5760
gtattcaaca tttccgtgtc gcccttattc ccttttttgc ggcattttgc cttcctgttt 5820
ttgctcaccc agaaacgctg gtgaaagtaa aagatgctga agatcagttg ggtgcacgag 5880
tgggttacat cgaactggat ctcaacagcg gtaagatcct tgagagtttt cgccccgaag 5940
aacgttttcc aatgatgagc acttttaaag ttctgctatg tggcgcggta ttatcccgta 6000
ttgacgccgg gcaagagcaa ctcggtcgcc gcatacacta ttctcagaat gacttggttg 6060
agtactcacc agtcacagaa aagcatctta cggatggcat gacagtaaga gaattatgca 6120
gtgctgccat aaccatgagt gataacactg cggccaactt acttctgaca acgatcggag 6180
gaccgaagga gctaaccgct tttttgcaca acatggggga tcatgtaact cgccttgatc 6240
gttgggaacc ggagctgaat gaagccatac caaacgacga gcgtgacacc acgatgcctg 6300
tagcaatggc aacaacgttg cgcaaactat taactggcga actacttact ctagcttccc 6360
ggcaacaatt aatagactgg atggaggcgg ataaagttgc aggaccactt ctgcgctcgg 6420

cccttccggc tggctggttt attgctgata aatctggagc cggtgagcgt gggtctcgcg 6480
gtatcattgc agcactgggg ccagatggta agccctcccg tatcgtagtt atctacacga 6540
cggggagtca ggcaactatg gatgaacgaa atagacagat cgctgagata ggtgcctcac 6600
tgattaagca ttggtaactg tcagaccaag tttactcata tatactttag attgatttaa 6660
aacttcattt ttaatttaaa aggatctagg tgaagatcct ttttgataat ctcatgacca 6720
aaatccctta acgtgagttt tcgttccact gagcgtcaga ccccgtagaa aagatcaaag 6780
gatcttcttg agatcctttt tttctgcgcg taatctgctg cttgcaaaca aaaaaaccac 6840
cgctaccagc ggtggtttgt ttgccggatc aagagctacc aactcttttt ccgaaggtaa 6900
ctggcttcag cagagcgcag ataccaaata ctgtccttct agtgtagccg tagttaggcc 6960
accacttcaa gaactctgta gcaccgccta catacctcgc tctgctaatc ctgttaccag 7020
tggctgctgc cagtggcgat aagtcgtgtc ttaccgggtt ggactcaaga cgatagttac 7080
cggataaggc gcagcggtcg ggctgaacgg ggggttcgtg cacacagccc agcttggagc 7140
gaacgaccta caccgaactg agatacctac agcgtgagct atgagaaagc gccacgcttc 7200
ccgaagggag aaaggcggac aggtatccgg taagcggcag ggtcggaaca ggagagcgca 7260
cgagggagct tccaggggga aacgcctggt atctttatag tcctgtcggg tttcgccacc 7320
tctgacttga gcgtcgattt ttgtgatgct cgtcaggggg gcggagccta tggaaaaacg 7380
ccagcaacgc ggccttttta cggttcctgg ccttttgctg gccttttgct cacatgttct 7440
ttcctgcgtt atcccctgat tctgtggata accgtattac cgcctttgag tgagctgata 7500
ccgctcgccg cagccgaacg accgagcgca gcgagtcagt gagcgaggaa gcggaagagc 7560
gcccaatacg caaaccgcct ctccccgcgc gttggccgat tcattaatgc agctggcacg 7620
acaggtttcc cgactggaaa gcgggcagtg agcgcaacgc aattaatgtg agttagctca 7680
ctcattaggc accccaggct ttacacttta tgcttccggc tcgtatgttg tgtggaattg 7740
tgagcggata acaatttcac acaggaaaca gctatgacca tgattacgcc aagcgcgcaa 7800
ttaaccctca ctaaagggaa caaaagctgg gtaccgggcc cccctcgag ctgcaggcgc 7860
agaactggta ggtatggaag atctatacat tgaatcaata ttggca 7906
<212> Type : DNA
<211> Length : 7906
SequenceName : SEQ ID NO 10
SequenceDescription :

Sequence
--------
<213> OrganismName :
<400> PreSequenceString :
attagccata ttagtcattg gttatatagc ataaatcaat attggctatt ggccattgca 60
tacgttgtat ctatatcata atatgtacat ttatattggc tcatgtccaa tatgaccgcc 120
atgttgacat tgattattga ctagttatta atagtaatca attacggggt cattagttca 180
tagcccatat atggagttcc gcgttacata acttacggta aatggcccgc ctggctgacc 240
gcccaacgac ccccgcccat tgacgtcaat aatgacgtat gttcccatag taacgccaat 300
agggactttc cattgacgtc aatgggtgga gtatttacgg taaactgccc acttggcagt 360
acatcaagtg tatcatatgc caagtccgcc ccctattgac gtcaatgacg gtaaatggcc 420
cgcctggcat tatgcccagt acatgacctt acgggacttt cctacttggc agtacatcta 480
cgtattagtc atcgctatta ccatggtgat gcggttttgg cagtacacca atgggcgtgg 540
atagcggttt gactcacggg gatttccaag tctccacccc attgacgtca atgggagttt 600
gttttggcac caaaatcaac gggactttcc aaaatgtcgt aataaccccg ccccgttgac 660
gcaaatgggc ggtaggcgtg tacggtggga ggtctatata agcagagctc gtttagtgaa 720
ccgtcagatc ctcactctct tccgcatcgc tgtctgcgag ggccagctgt tgggctcgcg 780
gttgaggaca aactcttcgc ggtctttcca gtactcttgg atcggaaacc cgtcggcctc 840
cgaacggtac tccgccaccg agggacctga gcgagtccgc atcgaccgga tcggaaaacc 900
tctcgagaaa ggcgtctaac cagtcacagt cgcaaggtag gctgagcacc gtggcgggcg 960
gcagcgggtg gcggtcgggg ttgtttctgg cggaggtgct gctgatgatg taattaaagt 1020
aggcggtctt gagacggcgg atggtcgagg tgaggtgtgg caggcttgag atccagctgt 1080
tggggtgagt actccctctc aaaagcgggc attacttctg cgctaagatt gtcagtttcc 1140
aaaaacgagg aggatttgat attcacctgg cccgatctgg ccatacactt gagtgacaat 1200
gacatccact ttgcctttct ctccacaggt gtccactccc aggtccaagt ttaaacgcta 1260
gcaccatggg ctggtcctgc atcatcctgt ttctggtggc caccgccacc ggcgtgcaca 1320
gccaggtgac cctgagagag agcggccctg ccctggtcaa gcccacccag accctgaccc 1380
tgacatgcac cttcagcggc ttcagcctga gcaccagcgg catgggcgtg agctggattc 1440
ggcagccccc tggcaagggc ctggaatggc tggcccacat ctactgggac gacgacaagc 1500
ggtacaaccc cagcctgaag agccggctga ccatcagcaa ggacaccagc cggaaccagg 1560
tggtgctgac catgaccaac atggaccccg tggacaccgc cacctactac tgcgccaggc 1620
gggagaccgt gttctactgg tacttcgacg tgtggggcag aggcaccccc gtgaccgtga 1680
gcagcggagg cggaggatcc caggtgcagc tggtgcagag cggagccgag gtgaagaagc 1740
ctggcgccag cgtcaaggtg tcctgcaagg ccagcggcta caccttcacc aactacggcc 1800
tgagctgggt gcggcaggcc cctgggcagg gcctggaatg gatgggctgg atcagcgcca 1860
acaacggcga caccaattac ggccaggaat tccagggccg ggtcaccatg accaccgaca 1920
ccagcaccag caccgcctac atggaactgc ggagcctgag aagcgacgac accgccgtgt 1980
actactgcgc cagggacagc agcagcagct gggccagatg gttcttcgac ctgtggggca 2040

ggggcaccct ggtgaccgtg agcagcgcca gcaccaaggg cccatccgtg ttccctctgg 2100
cccccttcctc caagtccacc tccggcggca ccgccgctct gggctgcctg gtgaaggact 2160
acttccctga gcctgtgacc gtgtcctgga actctggcgc cctgaccagc ggcgtgcaca 2220
ccttccctgc cgtgctgcag tcctccggcc tgtactccct gtcctccgtg gtgaccgtgc 2280
cttcctcctc cctgggcacc cagacctaca tctgtaacgt gaaccacaag ccctccaaca 2340
ccaaggtgga caagaaggtg gagcctaagt cctgtgacaa gacccacacc tgccctccct 2400
gccctgcccc tgagctgctg ggcggacctt ccgtgttcct gttccctcct aagcctaagg 2460
acaccctgat gatctcccgg acccctgagg tgacctgtgt ggtggtggac gtgtcccacg 2520
aggacccaga ggtgaagttc aactggtacg tggacggcgt ggaggtgcac aacgccaaga 2580
ccaagcctcg ggaggagcag tacaactcca cctaccgggt ggtgtctgtg ctgaccgtgc 2640
tgcaccagga ctggctgaac ggcaaagaat acaagtgtaa ggtctccaac aaggccctgc 2700
ctgcccccat cgagaaaacc atctccaagg ccaagggcca gcctagggag cctcaggtgt 2760
acaccctgcc tccctcccgg gacgagctga ccaagaacca ggtgtccctg acctgtctgg 2820
tgaagggctt ctacccttcc gacatcgccg tggagtggga gtccaacggc cagcctgaga 2880
acaactacaa gaccacccct cctgtgctgg actccgacgg ctccttcttc ctgtactcca 2940
agctgaccgt ggacaagtcc cggtggcagc agggcaacgt gttctcctgc tccgtgatgc 3000
acgaggccct gcacaaccac tacacccaga agtccctgtc cctgagccct ggctgaagct 3060
tggatccaga catgataaga tacattgatg agtttggaca aaccacaact agaatgcagt 3120
gaaaaaaatg ctttatttgt gaaatttgtg atgctattgc tttatttgta accattataa 3180
gctgcaataa acaagttaac aacaacaatt gcattcattt tatgtttcag gttcaggggg 3240
aggtgtggga ggttttttaa agcaagtaaa acctctacaa atgtggtatg gctgattatg 3300
atccggctgc ctcgcgcgtt tcggtgatga cggtgaaaac ctctgacaca tgcagctccc 3360
ggagacggtc acagcttgtc tgtaagcgga tgccgggagc agacaagccc gtcagggcgc 3420
gtcagcgggt gttggcgggt gtcggggcgc agccatgagg tcgactctag agccccgccg 3480
ccggacgaac taaacctgac tacggcatct ctgccccttc ttcgcggggc agtgcatgta 3540
atcccttcag ttggttggta caacttgcca actgggccct gttccacatg tgacacgggg 3600
ggggaccaaa cacaaagggg ttctctgact gtagttgaca tccttataaa tggatgtgca 3660
catttgccaa cactgagtgg ctttcatcct ggagcagact ttgcagtctg tggactgcaa 3720
cacaacattg cctttatgtg taactcttgg ctgaagctct tacaccaatg ctgggggaca 3780
tgtacctccc aggggcccag gaagactacg ggaggctaca ccaacgtcaa tcagaggggc 3840
ctgtgtagct accgataagc ggaccctcaa gagggcatta gcaatagtgt ttataaggcc 3900
cccttgttaa ccctaaacgg gtagcatatg cttcccgggt agtagtatat actatccaga 3960
ctaaccctaa ttcaatagca tatgttaccc aacgggaagc atatgctatc gaattagggt 4020
tagtaaaagg gtcctaagga acagcgatat ctcccacccc atgagctgtc acggttttat 4080
ttacatgggg tcaggattcc acgagggtag tgaaccattt tagtcacaag ggcagtggct 4140
gaagatcaag gagcgggcag tgaactctcc tgaatcttcg cctgcttctt cattctcctt 4200
cgtttagcta atagaataac tgctgagttg tgaacagtaa ggtgtatgtg aggtgctcga 4260
aaacaaggtt tcaggtgacg cccccagaat aaaatttgga cggggggttc agtggtggca 4320
ttgtgctatg acaccaatat aaccctcaca aaccccttgg gcaataaata ctagtgtagg 4380
aatgaaacat tctgaatatc tttaacaata gaaatccatg gggtggggac aagccgtaaa 4440
gactggatgt ccatctcaca cgaatttatg gctatgggca acacataatc ctagtgcaat 4500
atgatactgg ggttattaag atgtgtccca ggcagggacc aagacaggtg aaccatgttg 4560
ttacactcta tttgtaacaa ggggaaagag agtggacgcc gacagcagcg gactccactg 4620
gttgtctcta acacccccga aaattaaacg gggctccacg ccaatggggc ccataaacaa 4680
agacaagtgg ccactctttt ttttgaaatt gtggagtggg ggcacgcgtc agcccccaca 4740
cgccgccctg cggttttgga ctgtaaaata agggtgtaat aacttggctg attgtaaccc 4800
cgctaaccac tgcggtcaaa ccacttgccc acaaaaccac taatggcacc ccggggaata 4860
cctgcataag taggtgggcg ggccaagata ggggcgcgat tgctgcgatc tggaggacaa 4920
attacacaca cttgcgcctg agcgccaagc acagggttgt tggtcctcat attcacgagg 4980
tcgctgagag cacggtgggc taatgttgcc atgggtagca tatactaccc aaatatctgg 5040
atagcatatg ctatcctaat ctatatctgg gtagcatagg ctatcctaat ctatatctgg 5100
gtagcatatg ctatcctaat ctatatctgg gtagtatatg ctatcctaat ttatatctgg 5160
gtagcatagg ctatcctaat ctatatctgg gtagcatatg ctatcctaat ctatatctgg 5220
gtagtatatg ctatcctaat ctgtatccgg gtagcatatg ctatcctaat agagattagg 5280
gtagtatatg ctatcctaat ttatatctgg gtagcatata ctacccaaat atctggatag 5340
catatgctat cctaatctat atctgggtag catatgctat cctaatctat atctgggtag 5400
cataggctat cctaatctat atctgggtag catatgctat cctaatctat atctgggtag 5460
tatatgctat cctaatttat atctgggtag cataggctat cctaatctat atctgggtag 5520
catatgctat cctaatctat atctgggtag tatatgctat cctaatctgt atccgggtag 5580
catatgctat cctcatgcat atacagtcag catatgatac ccagtagtag agtgggagtg 5640
ctatcctttg catatgccgc cacctcccaa gggggcgtga attttcgctg cttgtccttt 5700
tcctgcatga gctccaattc gccctatagt gagtcgtatt acgcgcgctc actggccgtc 5760
gttttacaac gtcgtgactg ggaaaaccct ggcgttaccc aacttaatcg ccttgcagca 5820
catccccctt tcgccagctg gcgtaatagc gaagaggccc gcaccgatcg cccttcccaa 5880
cagttgcgca gcctgaatgg cgaatgggac gcgccctgta gcggcgcatt aagcgcggcg 5940
ggtgtggtgg ttacgcgcag cgtgaccgct acacttgcca gcgccctagc gcccgctcct 6000
ttcgctttct tcccttcctt tctcgccacg ttcgccggct ttccccgtca agctctaaat 6060
cgggggctcc ctttagggtt ccgatttagt gctttacggc acctcgaccc caaaaaactt 6120

gattagggtg atggttcacg tagtgggcca tcgccctgat agacggtttt tcgccctttg 6180
acgttggagt ccacgttctt taatagtgga ctcttgttcc aaactggaac aacactcaac 6240
cctatctcgg tctattcttt tgatttataa gggattttgc cgatttcggc ctattggtta 6300
aaaaatgagc tgatttaaca aaaatttaac gcgaatttta acaaaatatt aacgcttaca 6360
atttaggtgg cacttttcgg ggaaatgtgc gcggaacccc tatttgttta tttttctaaa 6420
tacattcaaa tatgtatccg ctcatgagac aataaccctg ataaatgctt caataatatt 6480
gaaaaaggaa gagtatgagt attcaacatt tccgtgtcgc ccttattccc ttttttgcgg 6540
cattttgcct tcctgttttt gctcacccag aaacgctggt gaaagtaaaa gatgctgaag 6600
atcagttggg tgcacgagtg ggttacatcg aactggatct caacagcggt aagatccttg 6660
agagttttcg ccccgaagaa cgttttccaa tgatgagcac ttttaaagtt ctgctatgtg 6720
gcgcggtatt atcccgtatt gacgccgggc aagagcaact cggtcgccgc atacactatt 6780
ctcagaatga cttggttgag tactcaccag tcacagaaaa gcatcttacg gatggcatga 6840
cagtaagaga attatgcagt gctgccataa ccatgagtga taacactgcg gccaacttac 6900
ttctgacaac gatcggagga ccgaaggagc taaccgcttt tttgcacaac atgggggatc 6960
atgtaactcg ccttgatcgt tgggaaccgg agctgaatga agccatacca aacgacgagc 7020
gtgacaccac gatgcctgta gcaatggcaa caacgttgcg caaactatta actggcgaac 7080
tacttactct agcttcccgg caacaattaa tagactggat ggaggcggat aaagttgcag 7140
gaccacttct gcgctcggcc cttccggctg gctggtttat tgctgataaa tctggagccg 7200
gtgagcgtgg gtctcgcggt atcattgcag cactggggcc agatggtaag ccctcccgta 7260
tcgtagttat ctacacgacg gggagtcagg caactatgga tgaacgaaat agacagatcg 7320
ctgagatagg tgcctcactg attaagcatt ggtaactgtc agaccaagtt tactcatata 7380
tactttagat tgatttaaaa cttcattttt aatttaaaag gatctaggtg aagatccttt 7440
ttgataatct catgaccaaa atcccttaac gtgagttttc gttccactga gcgtcagacc 7500
ccgtagaaaa gatcaaagga tcttcttgag atcctttttt tctgcgcgta atctgctgct 7560
tgcaaacaaa aaaaccaccg ctaccagcgg tggtttgttt gccggatcaa gagctaccaa 7620
ctctttttcc gaaggtaact ggcttcagca gagcgcagat accaaatact gtccttctag 7680
tgtagccgta gttaggccac cacttcaaga actctgtagc accgcctaca tacctcgctc 7740
tgctaatcct gttaccagtg gctgctgcca gtggcgataa gtcgtgtctt accgggttgg 7800
actcaagacg atagttaccg gataaggcgc agcggtcggg ctgaacgggg ggttcgtgca 7860
cacagcccag cttggagcga acgacctaca ccgaactgag atacctacag cgtgagctat 7920
gagaaagcgc cacgcttccc gaagggagaa aggcggacag gtatccggta agcggcaggg 7980
tcggaacagg agagcgcacg agggagcttc cagggggaaa cgcctggtat ctttatagtc 8040
ctgtcgggtt tcgccacctc tgacttgagc gtcgattttt gtgatgctcg tcaggggggc 8100
ggagcctatg gaaaaacgcc agcaacgcgg cctttttacg gttcctggcc ttttgctggc 8160
cttttgctca catgttcttt cctgcgttat cccctgattc tgtggataac cgtattaccg 8220
cctttgagtg agctgatacc gctcgccgca gccgaacgac cgagcgcagc gagtcagtga 8280
gcgaggaagc ggaagagcgc ccaatacgca aaccgcctct ccccgcgcgt tggccgattc 8340
attaatgcag ctggcacgac aggtttcccg actggaaagc gggcagtgag cgcaacgcaa 8400
ttaatgtgag ttagctcact cattaggcac cccaggcttt acactttatg cttccggctc 8460
gtatgttgtg tggaattgtg agcggataac aatttcacac aggaaacagc tatgaccatg 8520
attacgccaa gcgcgcaatt aaccctcact aaagggaaca aaagctgggt accgggcccc 8580
ccctcgagct gcaggcgcag aactggtagg tatggaagat ctatacattg aatcaatatt 8640
ggca 8644
<212> Type : DNA
<211> Length : 8644
SequenceName : SEQ ID NO 11
SequenceDescription :

Sequence
--------
<213> OrganismName :
<400> PreSequenceString :
attagccata ttagtcattg gttatatagc ataaatcaat attggctatt ggccattgca 60
tacgttgtat ctatatcata atatgtacat ttatattggc tcatgtccaa tatgaccgcc 120
atgttgacat tgattattga ctagttatta atagtaatca attacggggt cattagttca 180
tagcccatat atggagttcc gcgttacata acttacggta aatggcccgc ctggctgacc 240
gcccaacgac ccccgcccat tgacgtcaat aatgacgtat gttcccatag taacgccaat 300
agggactttc cattgacgtc aatgggtgga gtatttacgg taaactgccc acttggcagt 360
acatcaagtg tatcatatgc caagtccgcc ccctattgac gtcaatgacg gtaaatggcc 420
cgcctggcat tatgcccagt acatgacctt acgggacttt cctacttggc agtacatcta 480
cgtattagtc atcgctatta ccatggtgat gcggttttgg cagtacacca atgggcgtgg 540
atagcggttt gactcacggg gatttccaag tctccacccc attgacgtca atgggagttt 600
gttttggcac caaaatcaac gggactttcc aaaatgtcgt aataaccccg ccccgttgac 660
gcaaatgggc ggtaggcgtg tacggtggga ggtctatata agcagagctc gtttagtgaa 720
ccgtcagatc ctcactctct tccgcatcgc tgtctgcgag ggccagctgt tgggctcgcg 780
gttgaggaca aactcttcgc ggtctttcca gtactcttgg atcggaaacc cgtcggcctc 840
cgaacggtac tccgccaccg agggacctga gcgagtccgc atcgaccgga tcggaaaacc 900
tctcgagaaa ggcgtctaac cagtcacagt cgcaaggtag gctgagcacc gtggcgggcg 960

gcagcgggtg gcggtcgggg ttgtttctgg cggaggtgct gctgatgatg taattaaagt 1020
aggcggtctt gagacggcgg atggtcgagg tgaggtgtgg caggcttgag atccagctgt 1080
tggggtgagt actccctctc aaaagcgggc attacttctg cgctaagatt gtcagtttcc 1140
aaaaacgagg aggatttgat attcacctgg cccgatctgg ccatacactt gagtgacaat 1200
gacatccact ttgcctttct ctccacaggt gtccactccc aggtccaagt ttaaacgcta 1260
gcaccatggg ctggtcctgc atcatcctgt ttctggtggc caccgccacc ggcgtgcaca 1320
gcgacatcgt gatgacccag agccccgaca gcctggccgt gagcctgggc gagcgggcca 1380
ccatcaactg caaggccagc cagagcgtgg actacgacgg cgacagctac atgaactggt 1440
atcagcagaa gcccggccag ccccccaagc tgctgatcta cgccgccagc aacctggaaa 1500
gcggcgtgcc cgaccggttt agcggcagcg gctccggcac cgacttcacc ctgaccatca 1560
gcagcctgca ggccgaggac gtggccgtgt actactgcca gcagagcaac gaggaccccc 1620
ccaggtttgg cggcggaaca aaggtggaga tcaagggagg cggaggatcc agctacgtgc 1680
tgacccagcc ccccagcgtg agcgtggccc ctggcaagac cgcccggatc acctgcggcg 1740
gcaacatcat cggcagcaag ctggtgcact ggtatcagca gaagcccggc caggcccccg 1800
tgctggtgat ctacgacgac ggcgaccggc ccagcggcat ccccgagcgg ttcagcggca 1860
gcaacagcgg caacaccgcc accctgacca tcagccgggt ggaggccggc gacgaggccg 1920
actactactg ccaggtgtgg gacaccggca gcgaccccgt ggtgtttggc ggcggaacaa 1980
agctgaccgt gctgcgtacg gtggccgctc cttccgtgtt catcttccct ccctccgacg 2040
agcagctgaa gtccggcacc gcctccgtgg tgtgtctgct gaacaacttc taccctcggg 2100
aggccaaggt gcagtggaag gtggacaacg ccctgcagtc cggcaactcc caggagtccg 2160
tcaccgagca ggactccaag gacagcacct actccctgtc ctccaccctg accctgtcca 2220
aggccgacta cgagaagcac aaggtgtacg cctgtgaggt gacccaccag ggcctgtcca 2280
gccctgtgac caagtccttc aaccggggcg agtgctgaag cttggatcca gacatgataa 2340
gatacattga tgagtttgga caaaccacaa ctagaatgca gtgaaaaaaa tgctttattt 2400
gtgaaatttg tgatgctatt gctttatttg taaccattat aagctgcaat aaacaagtta 2460
acaacaacaa ttgcattcat tttatgtttc aggttcaggg ggaggtgtgg gaggtttttt 2520
aaagcaagta aaacctctac aaatgtggta tggctgatta tgatccggct gcctcgcgcg 2580
tttcggtgat gacggtgaaa acctctgaca catgcagctc ccggagacgg tcacagcttg 2640
tctgtaagcg gatgccggga gcagacaagc ccgtcagggc gcgtcagcgg gtgttggcgg 2700
gtgtcggggc gcagccatga ggtcgactct agagccccgc cgccggacga actaaacctg 2760
actacggcat ctctgcccct tcttcgcggg gcagtgcatg taatcccttc agttggttgg 2820
tacaacttgc caactgggcc ctgttccaca tgtgacacgg ggggggacca aacacaaagg 2880
ggttctctga ctgtagttga catccttata aatggatgtg cacatttgcc aacactgagt 2940
ggctttcatc ctggagcaga ctttgcagtc tgtggactgc aacacaacat tgcctttatg 3000
tgtaactctt ggctgaagct cttacaccaa tgctggggga catgtacctc ccaggggccc 3060
aggaagacta cgggaggcta caccaacgtc aatcagaggg gcctgtgtag ctaccgataa 3120
gcggaccctc aagagggcat tagcaatagt gtttataagg ccccettgtt aaccctaaac 3180
gggtagcata tgcttcccgg gtagtagtat atactatcca gactaaccct aattcaatag 3240
catatgttac ccaacgggaa gcatatgcta tcgaattagg gttagtaaaa gggtcctaag 3300
gaacagcgat atctcccacc ccatgagctg tcacggtttt atttacatgg ggtcaggatt 3360
ccacgagggt agtgaaccat tttagtcaca agggcagtgg ctgaagatca aggagcgggc 3420
agtgaactct cctgaatctt cgcctgcttc ttcattctcc ttcgtttagc taatagaata 3480
actgctgagt tgtgaacagt aaggtgtatg tgaggtgctc gaaaacaagg tttcaggtga 3540
cgcccccaga ataaaatttg gacggggggt tcagtggtgg cattgtgcta tgacaccaat 3600
ataaccctca caaacccctt gggcaataaa tactagtgta ggaatgaaac attctgaata 3660
tctttaacaa tagaaatcca tggggtgggg acaagccgta aagactggat gtccatctca 3720
cacgaattta tggctatggg caacacataa tcctagtgca atatgatact ggggttatta 3780
agatgtgtcc caggcaggga ccaagacagg tgaaccatgt tgttacactc tatttgtaac 3840
aaggggaaag agagtggacg ccgacagcag cggactccac tggttgtctc taacaccccc 3900
gaaaattaaa cggggctcca cgccaatggg gcccataaac aaagacaagt ggccactctt 3960
ttttttgaaa ttgtggagtg ggggcacgcg tcagccccca cacgccgccc tgcggttttg 4020
gactgtaaaa taagggtgta ataacttggc tgattgtaac cccgctaacc actgcggtca 4080
aaccacttgc ccacaaaacc actaatggca ccccggggaa tacctgcata agtaggtggg 4140
cgggccaaga taggggcgcg attgctgcga tctggaggac aaattacaca cacttgcgcc 4200
tgagcgccaa gcacagggtt gttggtcctc atattcacga ggtcgctgag agcacggtgg 4260
gctaatgttg ccatgggtag catatactac ccaaatatct ggatagcata tgctatccta 4320
atctatatct gggtagcata ggctatccta atctatatct gggtagcata tgctatccta 4380
atctatatct gggtagtata tgctatccta atttatatct gggtagcata ggctatccta 4440
atctatatct gggtagcata tgctatccta atctatatct gggtagtata tgctatccta 4500
atctgtatcc gggtagcata tgctatccta atagagatta gggtagtata tgctatccta 4560
atttatatct gggtagcata tactacccaa atatctggat agcatatgct atcctaatct 4620
atatctgggt agcatatgct atcctaatct atatctgggt agcataggct atcctaatct 4680
atatctgggt agcatatgct atcctaatct atatctgggt agtatatgct atcctaattt 4740
atatctgggt agcataggct atcctaatct atatctgggt agcatatgct atcctaatct 4800
atatctgggt agtatatgct atcctaatct gtatccgggt agcatatgct atcctcatgc 4860
atatacagtc agcatatgat acccagtagt agagtgggag tgctatcctt tgcatatgcc 4920
gccacctccc aagggggcgt gaattttcgc tgcttgtcct tttcctgcat gagctccaat 4980
tcgccctata gtgagtcgta ttacgcgcgc tcactggccg tcgttttaca acgtcgtgac 5040

tgggaaaacc ctggcgttac ccaacttaat cgccttgcag cacatccccc tttcgccagc 5100
tggcgtaata gcgaagaggc ccgcaccgat cgcccttccc aacagttgcg cagcctgaat 5160
ggcgaatggg acgcgccctg tagcggcgca ttaagcgcgg cgggtgtggt ggttacgcgc 5220
agcgtgaccg ctacacttgc cagcgcccta gcgcccgctc ctttcgcttt cttcccttcc 5280
tttctcgcca cgttcgccgg ctttccccgt caagctctaa atcgggggct ccctttaggg 5340
ttccgattta gtgctttacg gcacctcgac cccaaaaaac ttgattaggg tgatggttca 5400
cgtagtgggc catcgccctg atagacggtt tttcgccctt tgacgttgga gtccacgttc 5460
tttaatagtg gactcttgtt ccaaactgga acaacactca accctatctc ggtctattct 5520
tttgatttat aagggatttt gccgatttcg gcctattggt taaaaaatga gctgatttaa 5580
caaaaattta acgcgaattt taacaaaata ttaacgctta caatttaggt ggcacttttc 5640
ggggaaatgt gcgcggaacc cctatttgtt tatttttcta aatacattca aatatgtatc 5700
cgctcatgag acaataaccc tgataaatgc ttcaataata ttgaaaaagg aagagtatga 5760
gtattcaaca tttccgtgtc gcccttattc ccttttttgc ggcattttgc cttcctgttt 5820
ttgctcaccc agaaacgctg gtgaaagtaa aagatgctga agatcagttg ggtgcacgag 5880
tgggttacat cgaactggat ctcaacagcg gtaagatcct tgagagtttt cgccccgaag 5940
aacgttttcc aatgatgagc acttttaaag ttctgctatg tggcgcggta ttatcccgta 6000
ttgacgccgg gcaagagcaa ctcggtcgcc gcatacacta ttctcagaat gacttggttg 6060
agtactcacc agtcacagaa aagcatctta cggatggcat gacagtaaga gaattatgca 6120
gtgctgccat aaccatgagt gataacactg cggccaactt acttctgaca acgatcggag 6180
gaccgaagga gctaaccgct tttttgcaca acatggggga tcatgtaact cgccttgatc 6240
gttgggaacc ggagctgaat gaagccatac caaacgacga gcgtgacacc acgatgcctg 6300
tagcaatggc aacaacgttg cgcaaactat taactggcga actacttact ctagcttccc 6360
ggcaacaatt aatagactgg atggaggcgg ataaagttgc aggaccactt ctgcgctcgg 6420
cccttccggc tggctggttt attgctgata aatctggagc cggtgagcgt gggtctcgcg 6480
gtatcattgc agcactgggg ccagatggta agccctcccg tatcgtagtt atctacacga 6540
cggggagtca ggcaactatg gatgaacgaa atagacagat cgctgagata ggtgcctcac 6600
tgattaagca ttggtaactg tcagaccaag tttactcata tatactttag attgatttaa 6660
aacttcattt ttaatttaaa aggatctagg tgaagatcct ttttgataat ctcatgacca 6720
aaatccctta acgtgagttt tcgttccact gagcgtcaga ccccgtagaa aagatcaaag 6780
gatcttcttg agatcctttt tttctgcgcg taatctgctg cttgcaaaca aaaaaaccac 6840
cgctaccagc ggtggtttgt ttgccggatc aagagctacc aactcttttt ccgaaggtaa 6900
ctggcttcag cagagcgcag ataccaaata ctgtccttct agtgtagccg tagttaggcc 6960
accacttcaa gaactctgta gcaccgccta catacctcgc tctgctaatc ctgttaccag 7020
tggctgctgc cagtggcgat aagtcgtgtc ttaccgggtt ggactcaaga cgatagttac 7080
cggataaggc gcagcggtcg ggctgaacgg ggggttcgtg cacacagccc agcttggagc 7140
gaacgaccta caccgaactg agatacctac agcgtgagct atgagaaagc gccacgcttc 7200
ccgaagggag aaaggcggac aggtatccgg taagcggcag ggtcggaaca ggagagcgca 7260
cgagggagct tccaggggga aacgcctggt atctttatag tcctgtcggg tttcgccacc 7320
tctgacttga gcgtcgattt ttgtgatgct cgtcaggggg gcggagccta tggaaaaacg 7380
ccagcaacgc ggccttttta cggttcctgg ccttttgctg gccttttgct cacatgttct 7440
ttcctgcgtt atcccctgat tctgtggata accgtattac cgcctttgag tgagctgata 7500
ccgctcgccg cagccgaacg accgagcgca gcgagtcagt gagcgaggaa gcggaagagc 7560
gcccaatacg caaaccgcct ctccccgcgc gttggccgat tcattaatgc agctggcacg 7620
acaggtttcc cgactggaaa gcgggcagtg agcgcaacgc aattaatgtg agttagctca 7680
ctcattaggc accccaggct ttacacttta tgcttccggc tcgtatgttg tgtggaattg 7740
tgagcggata acaatttcac acaggaaaca gctatgacca tgattacgcc aagcgcgcaa 7800
ttaaccctca ctaaagggaa caaaagctgg gtaccgggcc ccccctcgag ctgcaggcgc 7860
agaactggta ggtatggaag atctatacat tgaatcaata ttggca 7906
<212> Type : DNA
<211> Length : 7906
SequenceName : SEQ ID NO 12
SequenceDescription :

## Claims

1. A polyvalent antibody comprising:
a. at least one first polypeptide which is a constant heavy chain; and
b. at least one second polypeptide which is a constant light chain,
wherein the first polypeptide is attached to two or more variable domains, and the second polypeptide is attached to two or more variable domains.

2. A dimeric polyvalent antibody comprising two identical polypeptide monomers,
wherein each polypeptide monomer comprises:
a. a first polypeptide which is a constant heavy chain; and
b. a second polypeptide which is a constant light chain,
wherein the first polypeptide is attached to two or more variable domains, and the second polypeptide is attached to two or more variable domains.

3. The polyvalent antibody according to any one of the claims 1 or 2, wherein the two or more variable domains attached to the first polypeptide are variable domains of a heavy and/or light chain, and the two or more variable domains attached to the second polypeptide are variable domains of a heavy and/or light chain.

4. The polyvalent antibody according any one of the claims 1 or 2, wherein the two or more variable domains attached to the first polypeptide are variable domains of a heavy chain, and the two or more variable domains attached to the second polypeptide are variable domains of a light chain.

5. The polyvalent antibody according to claims 1 to 4, wherein the two or more variable domains attached to the first polypeptide associate with the two or more variable domains attached to the second polypeptide to form two or more antigen binding sites.

6. The polyvalent antibody according to claim 5, wherein the two or more antigen binding sites are equal or different from each other.

7. The polyvalent antibody according to claims 1 to 6, which binds to two or more antigens.

8. The polyvalent antibody according to claims 1 to 7, wherein the two or more variable domains are linked to each other by one or more linkers.

9. The polyvalent antibody according to claims 1 to 8, wherein the constant heavy chain is selected from one of the following classes: γ, α*,* µ*,* ε and δ.

10. The polyvalent antibody according to claims 1 to 8, wherein the constant heavy chain is a γ chain.

11. The polyvalent antibody according to claims 1 to 10, wherein the constant light chain is selected from one of the following classes: *k* and λ.

12. The polyvalent antibody according to claims 1 to 11, wherein the amino acid sequence of the first polypeptide attached to two or more variable domains is selected from one of the following sequences: SEQ ID NO: 2 and SEQ ID NO: 6, and the amino acid sequence of the second polypeptide attached to two or more variable domains is selected from one of the following sequences: SEQ ID NO: 4 and SEQ ID NO: 8.

13. The polyvalent antibody according to claims 1 to 11, wherein the first polypeptide attached to two or more variable domains is encoded by a DNA sequence which is cloned into one of the following deposited plasmids: DSM19744 and DSM19746, and the second polypeptide attached to two or more variable domains is encoded by a DNA sequence which is cloned into one of the following deposited plasmids: DSM19745 and DSM19747.

14. The polyvalent antibody according to claims 1 to 13, which is tetravalent.

15. The polyvalent antibody according to claims 1 to 14, which is monospecific.

16. The polyvalent antibody according to claims 1 to 14, which is bispecific.

17. The polyvalent antibody according to claims 1 to 14, which is multispecific.

18. The polyvalent antibody according to claims 1 to 17, which allows a binding efficiency of at least 50% for the antigen.

19. The polyvalent antibody according to claims 1 to 18, wherein the two or more variable domains and/or the first and second polypeptides derive from a monoclonal or a polyclonal antibody.

20. The polyvalent antibody according to claims 1 to 19, which is murine, chimeric, synthetic, humanized or human.

21. A nucleic acid molecule encoding the first polypeptide attached to two or more variable domains of the polyvalent antibody according to claims 1 to 20.

22. The nucleic acid molecule according to claim 21, which is DNA.

23. The nucleic acid molecule according to claim 21, which is RNA.

24. The nucleic acid molecule according to claim 22, which has a nucleic acid sequence selected from one of the following sequences: SEQ ID NO: 1 and SEQ ID NO: 5.

25. The nucleic acid molecule according to claim 22, which sequence is cloned into one of the following deposited plasmids: DSM19744 and DSM19746.

26. A nucleic acid molecule encoding the second polypeptide attached to two or more variable domains of the polyvalent antibody according to claims 1 to 20.

27. The nucleic acid molecule according to claim 26, which is DNA.

28. The nucleic acid molecule according to claim 26, which is RNA.

29. The nucleic acid molecule according to claim 27, which has a nucleic acid sequence selected from one of the following sequences: SEQ ID NO: 3 and SEQ ID NO: 7.

30. The nucleic acid molecule according to claim 27, which sequence is cloned into one of the following deposited plasmids: DSM19745 and DSM19747.

31. A vector comprising the nucleic acid molecule according to claim 22, 24, 25, 27, 29 or 30.

32. A host cell comprising the nucleic acid molecule according to claim 22, 24, 25, 27, 29 or 30.

33. A host cell comprising the vectors according to claim 31.

34. The host cell according to claims 32 and 33, which is selected from the group consisting of: HEK293, CHO, NS-0 and COS-7.

35. A process for producing a polyvalent antibody according to claims 1 to 20 comprising:
a. transforming an eukaryotic host cell by incorporating a nucleic acid molecule according to claim 22, 24 or 25 and a nucleic acid molecule according to claim 27, 29 or 30;
b. cultivating the host cell under suitable conditions so that said nucleic acid molecules are expressed;
c. causing or allowing said first and second polypeptides to combine to form the polyvalent antibody; and
d. purifying the polyvalent antibody from the culture medium.

36. A process according to claim 35, wherein the host cell is selected from the group consisting of: HEK293, CHO, NS-0 and COS-7.

37. A pharmaceutical composition comprising an antibody according to any one of claims 1 to 20, 35 and 36, and a pharmaceutically acceptable excipient.

38. A pharmaceutical composition according to claim 37, which has a therapeutic use.

39. A pharmaceutical composition according to claim 38, which is used in the treatment of cancer, inflammation, autoimmune diseases, infections, cardiovascular diseases, respiratory diseases, neurological diseases or metabolic diseases.

40. A pharmaceutical composition according to claim 37, which has a diagnostic use.

41. Use of an antibody according to claims 1 to 20 in the manufacture of a medicament for the therapeutic and/or prophylactic treatment of cancer, inflammation, autoimmune diseases, infections, cardiovascular diseases, respiratory diseases, neurological diseases or metabolic diseases.

42. Use of an antibody according to claims 1 to 20 in the manufacture of a diagnostic composition to diagnose cancer, inflammation, autoimmune diseases, infections, cardiovascular diseases, respiratory diseases, neurological diseases or metabolic diseases.

43. An assay for an antigen using the polyvalent antibody according to any one of the claims 1 to 20, comprising:
a. incubating the polyvalent antibody and an antigen under conditions which permit binding of the antigen to the polyvalent antibody; and
b. detecting the antigen-polyvalent antibody binding.
